# EUROPÄISCHE PATENTSCHRIFT

(11) **EP 2 598 174 B1**
(45) Veröffentlichungstag und Bekanntmachung des Hinweises auf die Patenterteilung: **27.03.2019**
(21) Anmeldenummer: 10742798.1
(22) Anmeldetag: 30.07.2010
(51) Int. Cl.: A61K 49/00, A61B 5/0275

(54) **MESSVERFAHREN ZUR BESTIMMUNG EINER ORGANFUNKTION**
MEASUREMENT METHOD FOR DETERMINING AN ORGAN FUNCTION
PROCÉDÉ DE MESURE POUR LA DÉTERMINATION DE LA FONCTION D'UN ORGANE

(43) Veröffentlichungstag der Anmeldung: 05.06.2013
(73) Patentinhaber: SmartDyeLivery GmbH, 07743 Jena (DE)
(72) Erfinder: BAUER, Michael, 07743 Jena (DE); GONNERT, Falk A., 07743 Jena (DE); RECKNAGEL, Peter, 07743 Jena (DE)
(74) Vertreter: Ettmayr, Andreas
(86) Internationale Anmeldenummer: PCT/EP2010/061138
(87) Internationale Veröffentlichungsnummer: WO 2012/013247

(56) Entgegenhaltungen:
- EP-A2- 1 254 630
- WO-A1-2007/090468
- WO-A2-2009/016181
- US-A1- 2009 004 115
- US-A1- 2009 236 541
- LISY M-R ET AL: "Diagnosis of peritonitis using near-infrared optical imaging of in vivo labeled monocytes-macrophages", JOURNAL OF BIOMEDICAL OPTICS, SPIE - INTERNATIONAL SOCIETY FOR OPTICAL ENGINEERING, US, Bd. 11, Nr. 6, 1. November 2006 (2006-11-01), Seiten 64014-1, XP002548665, ISSN: 1083-3668, DOI: 10.1117/1.2409310 [gefunden am 2007-01-02]
- POLA, R. ET AL.: "HPMA-copolymer conjugates targeted to tum or endothelium using synthetic oligopeptides", JOURNAL OF DRUG TARGETING, Bd. 17, Nr. 10, 1. Dezember 2009 (2009-12-01), Seiten 763-776, XP008134629,
- SAKKA, S. ET AL.: "Prognostic Value of the Indocyanine Green Plasma Disappearance Rate in critically III Patients*", CHEST, Bd. 122, Nr. 5, 5. November 2002 (2002-11-05), Seiten 1715-1720, XP002630004,
- Dyomics GmbH: "Catalogue Fluorescent Dyes for Bioanalytical and Hightech Applications", 1. Oktober 2007 (2007-10-01), Dyomics GmbH, Jena , Germany, XP002630005, Seiten 1-85, das ganze Dokument

## Beschreibung

Die Erfindung betrifft ein Verfahren zur Datenerfassung für die Bestimmung einer Organfunktion, insbesondere der Leber- oder Nierenfunktion umfassend einem oder mehreren Polymethinfluoreszenzfarbstoffen mit einem Fluoreszenzemissionsmaximum gemessen in Ethanol von kleiner oder gleich 820 nm sowie die Verwendung der Farbstoffe als Marker zur Datenerfassung für die Bestimmung der Organfunktion. Die Erfindung beschreibt ebenfalls ein Kit zur Bestimmung einer Organfunktion mit einem Markerfarbstoff, sowie eine Vorrichtung zur Datenerfassung in dem Verfahren.

Es besteht ein zunehmender Bedarf an präzisen Messverfahren für die quantitative Bestimmung der Funktionstüchtigkeit eines Organs, insbesondere bei Organstörungen oder nach Transplantationen. Im Fokus stehen hierbei vor allem Leber und Niere.

Die Leber spielt aufgrund ihrer Funktion im Intermediärstoffwechsel, aber auch als Immunorgan, für den Verlauf und die Prognose kritisch kranker Patienten eine zentrale Rolle. Der Zelluntergang wird traditionell anhand der Freisetzung mehr oder minder spezifischer hepatozellulärer Enzyme (z.B. ASAT, ALAT, GLDH etc.) ins Plasma beurteilt ("statische" Tests).

Allerdings nimmt das Austreten von Enzymen aus der Leber bei Patienten, die an chronischer Hepatitis oder Zirrhose leiden, ab. Außerdem können Schwankungen oder hohe Werte auch von einer Person erhalten werden, wenn sich zum Zeitpunkt des Tests die Leberfunktion bereits von der Störung erholt hat, da es eine gewisse Zeit dauert, bis die ausgetretenen Enzyme aus dem Blut verschwinden. Somit sind quantitative Bestimmungen der Enzyme als Testverfahren zur Evaluierung des aktuellen Störungsgrads der Leberfunktion gewöhnlich nicht ausreichend. Traditionell ist Bilirubin der am häufigsten verwendete Indikator für die hepatische Dysfunktion. Allerdings kann dieser Indikator, wie auch die anderen statischen Tests, nur einen indirekten Hinweis auf die hepatische Funktion geben.

Im Gegensatz hierzu liefern dynamische Lebertests, wie zum Beispiel die Indozyaningrün-PDR (plasma disappearance rate/Plasmaschwundrate) direktere Angaben über den eigentlichen funktionellen Zustand der Leber zur Zeit der Untersuchung.

Die dynamischen Tests können wichtige Partialfunktionen des Hepatozyten liefern, wie der Intermediärstoffwechse! von Aminosäuren und Proteinen,- Harnstoffbiosynthese, Gluconeogenese, Metabolisierung von Xenobiotika oder Konjugationsund Exkretionsleistungen, die durch Gabe bestimmter Substrate im Rahmen der Testverfahren quantifiziert werden können.

In der europäischen Patentschrift EP 0 911 040 werden diagnostische Mittel zur Leberfunktion beschrieben. Hierin werden ¹³CO₂ markierte Atome über einen ¹³CO₂- Atemtest eingesetzt, um die Leberfunktion zu bestimmen. Ein ähnliches Verfahren wird auch in WO 02/075320 A2 präsentiert.

Allerdings erfordern dieses Tests die Gabe von markierten (z.B. radioaktiven) Tracern oder Substraten und sind aufgrund des hohen logistischen Aufwands oder der erforderlichen speziellen Laborverfahren in der klinischen Routine schwer oder gar nicht verfügbar.

Weitere Markersubstanzen, die im Urin oder Blutplasma gemessen werden können, sind 2,4,7-Triamino-6-phenylpteridin, wie in DE 301 6818 A1 beschrieben, oder Pentafluoroacetanilidoiminodiacetat (siehe EP 0 019 790).

Der Farbstoff ICG kann zur Bestimmung der Perfusionsverteilung (siehe DE 10 2005 044 531 A1) oder des Blutvolumens (siehe DE 41 30 931 C2) eingesetzt werden. Seit längerem wird ICG auch zur Untersuchung der Leberfunktion verwendet (siehe ICG-PDR in DE 689 21 947 T2); neuerdings auch über Pulsdetektionseinrichtungen verwendet (EP 0 399 482 B1).

In den letzten Jahren wurden die Erfassung der Indozyaningrün-Plasmaschwundrate (ICG-PDR_{ICG}) zur Beurteilung der Leberfunktion bei akuten oder chronischen Erkrankungen sowie nach Lebertransplantation als ergänzendes Messverfahren aufgrund von Praktikabilität und geringer Zeitintensität als bettseitiger Test im klinischen Alltag etabliert (Pulsion Medical Systems AG; DE 101 20 980 A1). Ebenfalls beschreibt Sakka et al., Chest, Bd. 122, Nr. 5, 5. Nov. 2002, S. 1715-1720 die Verwendung von Indocyaningrün (ICG) und der Messung der ICG "plasma disappearance rate (ICG-PDR)" zur Bestimmung der Leberfunktion in Intensivpatienten und als prognostischen Marker.

Wie jedes auf der Clearance eines Tracers beruhendes Messverfahren ist die PD-R_{ICG} von der Effizienz der Elimination [hepatozelluläre Leistung) und von der Perfusion abhängig.

Indozyaningrün (ICG) ist ein anionischer Farbstoff mit einem Absorptionsmaximum bei einer Wellenlänge von etwa 805 nm, der bei klinisch gebräuchlicher Dosierung eine vernachlässigbare Toxizität besitzt (siehe DE 699 21 151 T2). ICG wird nach dem derzeitigen Kenntnisstand extrahepatisch weder metabolisiert noch eliminiert und unterliegt keinem enterohepatischen Kreislauf. Nach intravenöser Injektion (0,1-0,5 mg/kg KG) ist ICG innerhalb kurzer Zeit vollständig an Plasmaproteine gebunden und erscheint nach etwa 8 min unkonjugiert in der Galle. Hepatische Aufnahme und Elimination von ICG sind passive, respektive aktive Prozesse, die von Herzzeitvolumen, sinusoidaler Perfusion, Membrantransport und sekretorischer Kapazität der Leber abhängen. Die Messung kann mittlerweile nichtinvasiv durch transkutane Bestimmung der PDR_{ICG} mittels Pulsdensitometrie erfolgen.

Verschiedene klinische Untersuchungen an unterschiedlichen Patientenkollektiven haben gezeigt, dass dieses Verfahren eine hepatozelluläre Dysfunktion bereits zu einem frühen Zeitpunkt detektieren kann, darüber hinaus gut mit der Uberlebensrate korreliert und als prognostischer Marker gegenüber den herkömmlichen statischen Laborparametern deutliche Vorteile besitzt.

Der Transport von ICG in die Galle beinhaltet mindestens 3 Schritte: die Aufnahme in den Hepatozyten durch die sinusoidale Membran, die Passage durch den Hepatozyten und die Exkretion über die canaliculäre Membran. Diese Transportschritte wurden jedoch nur anhand von kinetischen Studien charakterisiert. Bislang existie ren wenige Daten, die Aufschluss über das Verhältnis von PDR_{ICG} und biliärer ICG-Sekretion unter pathophysiologischen Bedingungen wie Sepsis, Schock oder Endotoxinämie geben. Eine aktuelle Studie untersuchte das Verhältnis von PDR_{ICG} und biliärer ICG-Exkretion unter Endotoxinämie an Schweinen. Während die PDR_{ICG} auch nach 12 Stunden Endotoxinämie nahezu unverändert war, kam es dennoch zu einer signifikanten Abnahme der biliären ICG-Sekretion sowie des kumulativen Galleflusses, ohne dass bei insgesamt hyperdynamer Kreislaufsituation wesentliche Änderungen der Leberperfusion oder des Sauerstoffverbrauchs der Leber zu verzeichnen waren.

Die Aufnahme von ICG aus den Lebersinusoiden erfolgt durch die basolaterale (sinusoidaie) Plasmamembran des Hepatozyten. Im Gegensatz dazu erfolgt die Exkretion von ICG über die apikale (canaliculäre) Membran - einen hochgradig energieabhängigen Transportmechanismus, der überaus sensitiv gegenüber Endotoxin ist. Somit könnte unter bestimmten pathophysiologischen Bedingungen wie der Sepsis zwar ICG in die Hepatozyten aufgenommen, jedoch nicht über die Galle wieder eliminiert werden und damit die Bestimmung der PDR_{ICG} zur Therapiesteuerung und Prognoseeinschätzung unzuverlässig werden.

Der totale Umsatz von ICG setzt sich aus zwei Komponenten zusammen: die Aufnahme in die Hepatozyten aus dem Blut und der Transport von ICG aus den Leberzellen in das Galleexkret. Durch gängige PDR-Messungen wird in erster Linie der erste Schritt gemessen. Allerdings sind diese beiden Schritte im Falle einer Akkumulation von ICG in den Hepatozyten entkoppelt und damit voneinander unabhängig. Damit steht die Gesamtorganfunktion nicht mehr im linearen Zusammenhang mit der gemessenen PDR, weil die Abnahme im Blut vorwiegend auf den ersten Schritt zurückgeführt wird und nicht mehr ein Maß für die Exkretion in die Gallenflüssigkeit darstellt.

Bei Verzicht auf eine Bestimmung der prä- und posthepatischen Plasmakonzentrationen und lediglich Messung der systemischen PDR_{ICG} können naturgemäß beide Einflussgrößen (hepatozelluläre fraktionelle Clearance vs. Leberblutfluss) nicht diskriminiert werden.

Bei der klinischen Beurteilung der exkretorischen Nierenfunktion kommt die größte Bedeutung der glomerulären Filtration zu. Der Goldstandard zur Messung der glomerulären Filtrationsrate (GFR) ist die Inulinclearance, die jedoch methodisch für den Routineeinsatz zu aufwändig ist. In der klinischen Routine kann mit guter Näherung die Kreatininclearance als Maß für die GFR bestimmt werden. Allerdings unterliegt diese den Störeinflüssen der reatininbestimmung und ist durch Fehler während der Urinsammeiperiode zusätzlich fehlerbehaftet. Da Kreatinin bei stark eingeschränkter GFR auch tubulär sezerniert wird, kann zudem die "wahre" GFR um bis zu 100% überschätzt werden. Methoden zur kontinuierlichen Überwachung der GFR im Sinne eines Monitoring, wie es für hämodynamische und respiratorische Parameter möglich ist, existieren nur experimentell.

Im Allgemeinen ist es daher die Aufgabe der jetzigen Erfindung, ein verbessertes Verfahren zur Bestimmung einer Organfunktion, vor allem der Leber oder der Niere bereitzustellen, welches die oben beschriebenen Probleme umgeht.

Insbesondere ist die Aufgabe der vorliegenden Erfindung, ein Verfahren bereitzustellen, das eine klinische Einschätzung der am Blutfluss teilnehmenden intakten Hepatozytenmasse, also eine gute Einschätzung der Leberleistung und ihrer therapeutischen Beeinflussbarkeit erlaubt.

Dieses Verfahren soll vor allem eine genaue Diagnose zur Prognose im Zustand der hepatischen Dysfunktion der Leber aufgrund von Sepsis oder anderen Störungen liefern.

Außerdem ist es ein Ziel der Erfindung, eine verbesserte Bildgebung und genauer quantifizierbare Messung der verwendeten Markersubstanzen zu ermöglichen.

Es sollen Verwendungen, bereitgestellt werden, welche in Analogie die oben beschriebenen Probleme lösen und Vorteile aufweisen.

Überraschend lassen sich die oben beschriebenen Probleme mit den im Folgenden beschriebenen erfinderischen Verwendungen und Verfahren lösen.

Insbesondere befasst sich die vorliegende Erfindung mit der Verwendung eines oder mehrerer Polymethinfluoreszenzfarbstoffe mit einem Fluoreszenzemissionsmaximum, gemessen in Ethanol, von kleiner oder gleich 820 nm als Marker zur Datenerfassung für die Bestimmung einer Organfunktion.

Die vorliegende Erfindung betrifft daher in einem ersten Aspekt einen oder mehrere Polymethinfluoreszenzfarbstoffe zur Verwendung als Marker zur Datenerfassung für die Bestimmung einer Organfunktion *in vivo*, wobei der eine oder die mehreren Polymethinfluoreszenzfarbstoffe ein Fluoreszenzemissionsmaximum, gemessen in Ethanol, von kleiner oder gleich 820 nm aufweist, und wobei das Organ, dessen Funktion bestimmt wird, die Leber und/oder die Niere ist.

Wie oben beschriebenen ist der Einsatz von langwellig emittierenden Polymethinfluoreszenzfarbstoffen zur Organdiagnostik bereits bekannt, so auch z.B. ICG. Polymethinfarbstoffe sind konjugierte Polyene, in denen ein Elektronenakzeptor über eine ungeradzahlige Kette von Methin-Gruppen mit dem Elektronen-Donor verknüpft ist.

Es wurde nun festgestellt, dass Fluoreszenzfarbstoffe, die unterhalb von 820 nm emittieren, zu einer besseren Bildgebung bei der Fluoreszenzdetektion führen. Damit lassen sich genauere Angaben zu den Farbstoffkonzentrationen im Blutserum, in anderen Flüssigkeitsmedien oder in den Organgeweben des Körpers, z.B. bei der Bestimmung der Organ-Perfusion, ermitteln. Eine bessere Auflösung bei den üblichen Bildgebungsverfahren ist insbesondere für ImagingVerfahren oder in der Intravitalmikroskopie von Vorteil.

Außerdem können die mit kürzeren Wellenlängen emittierenden Farbstoffe in Kombination mit weiteren bereits bekannten Farbstoffen wie ICG verwendet werden, da sich die Emissionswellenlängenbereiche mit verschiedenen Filtern (z.B. Strahlteiler) trennen lassen und über mehrere Detektionskanäle gleichzeitig oder konsekutiv erfasst werden können.

Der Farbstoff ICG weist zwischen dem Donor und dem Akzeptor (jeweils Stickstoff) 9 Kohlenstoffatome und 7 Methingruppen auf. Bevorzugt ist, dass die Farbstoffe nach der Erfindung weniger als neun Kohlenstoffatome zwischen dem Donor und dem Akzeptor der konjugierten Chromophorenkette des Fluoreszenzfarbstoffs aufweisen, besonders bevorzugt sind sieben oder fünf Kohlenstoffatome. Am bevorzugten weist die Chromophorenkette 7, 5 oder 3 Methingruppen auf.

Die Erfindung betrifft in einem zweiten Aspekt einen oder mehrere Polymethinfluoreszenzfarbstoffe mit einem Fluoreszenzemissionsmaximum, gemessen in Ethanol, von kleiner oder gleich 820 nm zur Verwendung in einem Verfahren zur Datenerfassung für die Bestimmung einer Organfunktion umfassend die Schritte:
a) Radiative Anregung *in vivo* von dem einen oder den mehreren Polymethinfluoreszenzfarbstoffen mit einem Fluoreszenzemissionsmaximum gemessen in Ethanol von kleiner oder gleich 820 nm, als Marker im Blut, in einem Gewebe oder Organ und/oder in der Galle oder Urin, mit einer Strahlungsquelle,
b) Detektion der Fluoreszenzemission des einen oder mehrerer Polymethinfarbstoffe mit einer Detektionsvorrichtung,
c) Erfassung der Daten der gemessenen Fluoreszenzintensität mit einer Datenerfassungsvorrichtung,
wobei das Organ, dessen Funktion bestimmt wird, die Leber und/oder die Niere ist.

In einem dritten Aspekt betrifft die Erfindung ein Verfahren zur Datenerfassung für die Bestimmung einer Organfunktion *in vitro* / *ex vivo* umfassend die Schritte:
a) Radiative Anregung von dem einen oder den mehreren Polymethinfluoreszenzfarbstoffen mit einem Fluoreszenzemissionsmaximum gemessen in Ethanol von kleiner oder gleich 820 nm, als Marker im Blut, in einem Gewebe oder Organ und/oder in der Galle oder Urin, mit einer Strahlungsquelle,
b) Detektion der Fluoreszenzemission des einen oder mehrerer Polymethinfarbstoffe mit einer Detektionsvorrichtung,
c) Erfassung der Daten der gemessenen Fluoreszenzintensität mit einer Datenerfassungsvorrichtung,
wobei das Organ, dessen Funktion bestimmt wird, die Leber und/oder die Niere ist.

Die eingesetzten Farbstoffe erlauben eine genauere Repräsentation der gemessenen Organfunktion. Zudem weisen sie eine geringere Tendenz zur Akkumulation in dem Organgewebe auf.

Daher stellen sie nicht nur die Perfusionsbewegung in das Organ dar, sondern geben außerdem die tatsächliche Exkretionsleistung des Organs besser wieder. Damit eignet sich das obige Verfahren insbesondere zur Bestimmung von Messwerten und Parametern, die für die Organfunktionsdiagnostik verwendet werden können.

Als bevorzugte Anregungsquelle für die radiative Anregung (Bestrahlung mit Licht) kann eine Laserdiode dienen. Um ein Ausbleichen des Farbstoffs zu verhindern, ist bei manchen Imagingverfahren die Leistung der Quelle bevorzugt kleiner gleich 1 mW/cm².

In Schritt c) werden die Daten bevorzugt als Funktion der Zeit gemessen. Hieraus lässt sich ein Graph der Fluoreszenz über die Zeit herleiten.

Besonders vorteilhaft ist die Messung der Fluoreszenz im Blutplasma. So lässt sich die Plasma disappearance rate (PDR) des Farbstoffs bestimmen. Es kann die Clearance-Funktion des Farbstoffs aus dem Blut oder dem Organgewebe bestimmt werden.

Günstig ist, dass die Leberfunktion nun "ganzheitlich" erfasst wird, da auch die exkretorische Leistung beurteilt werden kann.

Weiterhin bevorzugt ist die anschließende Speicherung der Daten der gemessenen Fluoreszenzintensitätsfunktion mit einer Datenspeicherungsvorrichtung.

Günstig ist auch die Bearbeitung der Daten und/oder Ausgabe der Daten an einer Datenausgabevorrichtung, z.B. einem Display, einem Ausdruck oder einem Speichermedium.

Vorzugsweise werden die erfassten Daten anschließend mit Normwerten verglichen. Im Anschluss hieran kann in einem weiteren Schritt eine (signifikante) Abweichung von den Normwerten festgestellt werden. Schließlich ist es hiernach optional auch möglich, aus diesem Analyseschritt mit der Hilfe eines Auswerteprogramms oder mit dem Erfahrungsschatz eines trainierten neuronalen Netzes eine Diagnose und/oder Zuordnung des Datensatzes zu einem pathologischen Befund festzustellen.

Der Farbstoff kann schließlich in bevorzugten Ausführungen des Verfahrens in einem ersten Schritt verabreicht werden. Noch bevorzugter erfolgt die Verabreichung von einen oder mehreren Polymethinfluoreszenzfarbstoffen durch intra-venöse Injektion in die Blutbahn. Alternativ kann der Farbstoff über einen bereits bestehenden Katheter verabreicht werden, wodurch ein zusätzlicher Eingriff am Körper entfällt. Bevorzugt wird die Verabreichung an einer Stelle vorgenommen, die nicht weiter als 50 cm von dem zu untersuchenden Gefäßgebiet oder Organ liegt.

Die Dosierung des Farbstoffs variiert mit der Körpermasse und den Anforderungen an die Messdetektion. Typischerweise werden 0,05 bis 10 mg/kg pro Messung eingesetzt, Besonders bevorzugt sind 0,1 bis 0,5 mg/kg oder bis 1,25 mg/kg pro Messung, am bevorzugtesten sind 0,25 mg/kg oder 0,45 mg/kg pro Messung. Wesentlich höhere Werte werden nicht empfohlen, da hier die an sich geringe Zytotoxizität ansteigen kann. Aus Figur 10 lässt sich entnehmen, dass im Vergleich der Zytotoxizität zwischen ICG und DY635 letzterer Farbstoff eine sehr geringe Zytotoxizität aufweist und keine geringere Viabilität als ICG zeigt.

In einer vorteilhaften Ausführung erfolgt die Fluoreszenzdetektion in der obigen Verwendung oder Verfahren *in vitro.* Dabei können die Messwerte nach Probenentnahme in einem Labor bestimmt werden.

Bevorzugt wird bei den erfindungsgemäßen Verwendungen oder Verfahren die Fluoreszenzdetektion am Blut, Gewebe oder Organ und/oder der Galle oder Urin eines Tieres vorgenommen; vorzugsweise ist dieses Tier ein Säugetier, Nager oder Homo Sapiens.

Ebenfalls offenbart wird ein Kit zur Bestimmung einer Organfunktion mit einem Markerfarbstoff umfassend:
ein oder mehrere Polymethinfluoreszenzfarbstoffe mit einem Fluoreszenzemissionsmaximum, gemessen in Ethanol, von kleiner oder gleich 820 nm, welche in einer physiologisch verträglichen Verabreichungsform vorliegen,
einer Fluoreszenzdetektionsvorrichtung,
und einer Fluoreszenzdatenerfassungsvorrichtung.

Dieses Kit hat den Vorteil, in kompakter Weise die Mittel für eine zügige Organfunktionsdiagnostik bereitzustellen.

Typischerweise umfasst das Kit genug Vorrat für ein bis zwei oder mehrere Dosierungen des Farbstoffs. Mehrfachdosiereinheiten können bis zu 100, 200 oder 500 Dosierungen enthalten. In diesem Fall sind die weiteren Bestandteile des Kits wiederverwertbar.

Das Kit kann zur Messung der PDR eingesetzt werden. Dabei ist zweckmäßig der Detektor ein Einmalsensor für die Messung am Ohrläppchen.

In einer offenbarten Weiterbildung umfasst der Kit zudem einen oder mehrere der folgenden Elemente: eine Verabreichungsvorrichtung (bevorzugt eine Injektionsnadel oder ein zentraler oder peripherer Katheter), eine Datenspeicherungsvorrichtung und eine Datenausgabevorrichtung.

In einer besonderen Ausführungsform der Erfindung liegen ein oder mehrere Polymethinfluoreszenzfarbstoffe in einer physiologisch verträglichen und intravenös injizierbaren Lösung vor. Diese können ganz besonders bevorzugt über einen bereits bestehenden Katheter verabreicht werden, so dass keine weiteren Eingriffe am Körper notwendig sind.

Weiterhin offenbart umfasst das Kit eine Dosiereinheit, welches eine Durchstechflasche sein kann, in der die Farbstofflösungen aufbewahrt werden können. In einem solchen Fall, umfasst das Kit Portionierungen enthaltend 25 mg Farbstoff auf ml Lösungsmittel, oder vorzugsweise 50 mg Farbstoff auf 10 ml Lösungsmittel, vorzugsweise Wasser.

In einer offenbarten Ausführung enthält dasKit den Farbstoff als Pulver in individuellen Dosiereinheiten, sowie optional die entsprechende Menge an Lösungsmittel, um die physiologische Lösung vor Ort herzustellen. Diese Variante hat den Vorteil einer besseren Lagerstabilität der Kitbestandteile.

Bei der Verwendung des Kits ist eine sterile Handhabung wichtig. Vorzugsweise liegt dem Kit eine Beschreibungsanleitung bei.

Die oben beschriebenen erfinderischen Verwendungen oder Verfahren zur Bestimmung einer Organfunktion lassen sich insbesondere bevorzugt dann einsetzen, wenn das Organ, dessen Funktion bestimmt wird, die Leber und/oder die Niere ist. Die eingesetzten Farbstoffe der Erfindung weisen in diesen Organen insbesondere den Vorteil auf, die eigentliche Organfunktion besser zu repräsentieren, weil mit ihnen die tatsächliche Exkretionsleistung des Organs genauer wiedergegeben wird.

Besonders bevorzugt sind die Verwendungen oder Verfahren nach der Erfindung, wobei das Fluoreszenzemissionsmaximum des einen oder der mehreren Polymethinfluoreszenzfarbstoffe gemessen in Ethanol kleiner gleich 810 nm ist.

Noch bevorzugter ist das Fluoreszenzemissionsmaximum kleiner gleich 780 nm oder 740 nm, am aller bevorzugtesten kleiner gleich 680 nm. Die Verwendung von Farbstoffen mit einem Fluoreszenzemissionsmaximum kleiner gleich 550 nm kann für besondere Verfahren geeignet sein, insbesondere in Kombination mit einem Farbstoff mit einem Fluoreszenzemissionsmaximum von größer gleich 750 nm.

Bevorzugt liegt das Absorptionsmaximum des Farbstoffs bei 580 nm bis 690 nm. Besonders bevorzugt ist ein Absorptionsmaximum von 600 nm bis 670 nm.

Um das Ausbleichen des Farbstoffs und damit eine Verzerrung der Messergebnisse zu verhindern, sollte der Farbstoff eine hohe Ausbleichstabilität aufweisen. Ebenfalls sinnvoll ist es, Farbstoffe mit einer hohen Fluoreszenzquantenausbeute zu verwenden, da diese bei entsprechender Anregung eine stärkere Leuchtkraft aufweisen.

Bevorzugte Ausführungsformen der Erfindung umfassen daher Verwendungen oder Verfahren, wobei der Farbstoff ein Benzopyrylium-basiertes Hemicyanin oder ein Indodicarbocyanin-Chromophor ist.

Bevorzugt ist die Zahl der Kohlenstoffatome zwischen der Donor und der Akzeptorgruppe am Farbstoff kleiner gleich 7. Dies hat den Effekt, dass der Farbstoff eine bessere Photo- und Lagerstabilität aufweist. Im Vergleich zu längerkettigen Chromophoren wie ICG bietet die kürzere konjugierte Doppelbindungskette des Farbstoffs mit ihrer kürzeren und weniger ausgeprägten Elektronenwolke weniger Angriffspositionen für elektrophile oder nukleophile Reagenzien. Dies erhöht die Fotostabilität der erfindungsgemäßen Farbstoffe, was bei quantitativen Imagingund Fluoreszenzdetektionsverfahren zu einer genaueren Messdatenerhebung führt.

Insbesondere können der eine oder die mehreren Farbstoffe der Erfindung ein unsymmetrisches Polymethin, aufweisend einer substituierten omega-(Benz[b]pyran-4-yliden)alk-1-enyl)-Einheit der allgemeinen Struktur 1 sein: 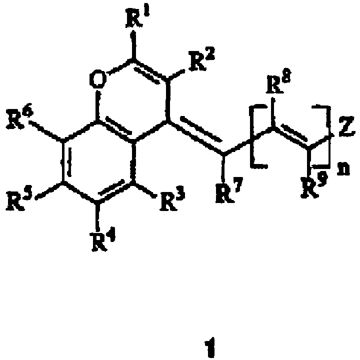 mit Z als substituiertes Benzooxazol-, Benzothiazol-, 2,3,3-Trimethylindolenin-, 2,3,3-Trimethyl-4,5-benzo-3H-indolenin-, 2- und 4-Picolin-, Lepidin-, Chinaldin sowie 9-Methylacridinderivat der allgemeinen Strukturformeln 2A oder 2B oder 2C 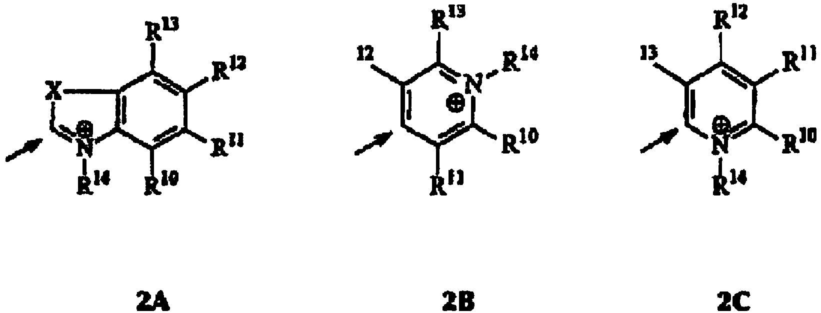 wobei
- X für ein Element aus der Gruppe O, S, Se oder das Strukturelement N-alkyl oder C(alkyl)₂ steht,
- n für die Zahlenwerte 1, 2 oder 3 steht,
- R¹ - R¹⁴ gleich oder unterschiedlich sind und Wasserstoff, ein oder mehrere Alkyl-, oder Aryl-, Heteroaryl oder heterocycloaliphatische Reste, eine Hydroxy- oder Alkoxygruppe, eine alkylsubsituierte oder cyclische Aminfunktion sein können und/oder zwei orthoständige Reste, z.B. R¹⁰ und R¹¹, R⁴ und R⁵ und/oder R⁶ und R⁵ zusammen einen weiteren aromatischen Ring bilden können,
- mindestens einer der Substituenten R¹ - R¹⁴ einen solubilisierenden bzw. ionisierbaren bzw. ionisierten Substituenten, wie Cyclodextrin, Zucker, SO₃⁻, PO₃²⁻, COO⁻, oder NR₃⁺ darstellen kann, der die hydrophilen Eigenschaften dieser Farbstoffe bestimmt, wobei dieser Substituent auch über eine Spacergruppe am Farbstoff angebunden sein kann, und
- R¹ einen Substituenten darstellt, der in alpha-Position zum Pyran-Ring ein quartäres C-Atom aufweist, bevorzugt sind t-Butyl und Adamantyl.

In vorteilhaften Ausführungen der Verwendungen oder Verfahren nach der Erfindung sind die Farbstoffe derart mit Substituenten versehen, dass sie jeweils auf verschiedene Organe angepasst sind. So hat sich gezeigt, dass nierengängige Farbstoffe eine nicht zu geringe Wasserlöslichkeit aufweisen sollten.

Daher sind die Polymethinfluoreszenzfarbstoffe für die Nierenfunktionsbestimmung dadurch gekennzeichnet, dass der eine oder die mehreren Farbstoffe in Wasser löslich sind aber in DMF oder DMSO unlöslich sind. Diese Farbstoffe sind tendenziell hydrophil.

Im Gegensatz hierzu sind die Polymethinfluoreszenzfarbstoffe nach den erfinderischen Verwendungen oder Verfahren für die Leberfunktionsbestimmung dadurch gekennzeichnet, dass der eine oder die mehreren Farbstoffe in DMF oder DMSO löslich und in Wasser unlöslich sind. Diese Farbstoffe sind tendenziell fettlöslich und hydrophob.

Insbesondere weisen der eine oder die mehreren Farbstoffe bevorzugt in physiologischer Lösung eine neutrale Ladung auf oder liegen höchstens einfach geladen vor und/oder weisen bevorzugt höchstens zwei solubilisierende bzw. ionisierbare bzw. ionisierte Substituenten auf. Diese Weiterbildungen sind besonders für die Leberfunktionsbestimmung geeignet.

In anderen Weiterbildungen der Erfindung liegen der eine oder die mehreren Farbstoffe in physiologischer Lösung mindestens zweifach geladen vor und/oder weisen bevorzugt mindestens drei oder vier solubilisierende bzw. ionisierbare bzw. ionisierte Substituenten auf.

Die genannten solubilisierenden bzw. ionisierbaren bzw. ionisierten Substituenten sind hierbei vor allem bevorzugt Sulfonsäuregruppen, Carbonsäuregruppen oder Ammoniumgruppen.

Besonders bevorzugt ist, dass der eine oder die mehreren Farbstoffe nach der Erfindung ausgewählt sind aus der Gruppe umfassend:
DY630, DY631, DY632, DY633, DY634, DY635, DY636, DY647, DY648, DY649, DY650, DY651 und DY652, sowie DY590, DY548, DY495, DY405.

Diese Farbstoffe weisen dabei bevorzugt eine Absorption im roten Spektralbereich auf. Allerdings sind auch Rot, Grün oder Blau absorbierende Farbstoffe verwendbar.

Ganz besonders bevorzugt ist der Farbstoff DY635, also:
1-(5-Carboxypentyl)-3,3-dimethyl-2-[3-(11-(2,2-dimethylethyl)-1H,2H,3H,5H,6H,7H pyrano[2,3-f]pyrido[3,2,1-ij]chinolin-9-yliden)-1-propenyl]-3H-indolium-5-sulfonat
   oder
10-tert-Buryl-8-{(E)-3-[1-(5-carboxy-pentyl)-3,3-dimethyl-5-sulfo-1,3-dihydro-indol(2E)-ylidene]-propenyl)-2,3,5,6-tertrahydro-1H,4H-11-oxonia-3a-aza-benzo[de]anthracene wie unten gezeigt:

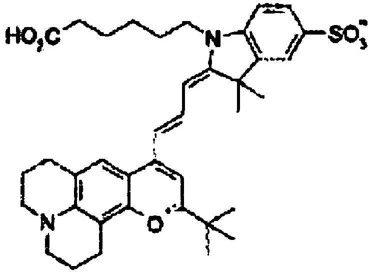

Bevorzugte ist auch DY647, also:
2-{(1E,3E)-5-[3-(3-Carboxy-propyl)-1-ethyl-3-methyl-5-sulfo-1,3-dihydro-indol-(2E)-ylidene]-penta-1,3-dienyl)-1-ethyl-3,3-dimefhyl-5-sulfo-3H-indolium 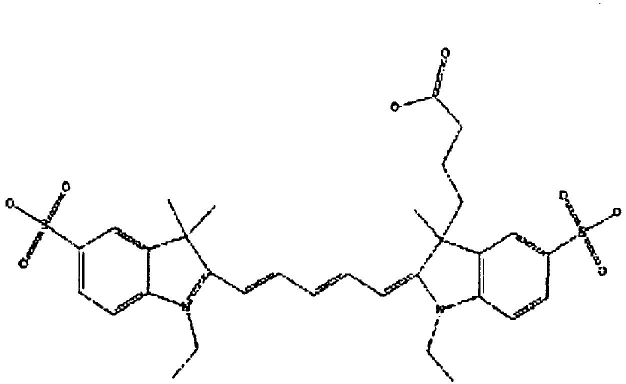 oder auch DY652, also :
2-*tert*-Butyl-4-{(E)-3-[3-(3-carboxy-propyl)-3-methyl-5-sulfo-1-(3-sulfo-propyl)-1,3-dihydro-indol-(2Z)-ylidene]-propenyl)-5,7,7-trimethyl-8-(3-sulfo-propyl)-7,8-dihydro-1-oxanα-8-αzα-αnthracene wie unten gezeigt:

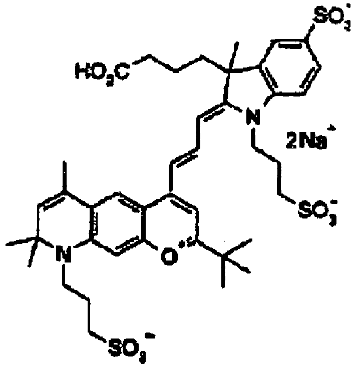

Mit diesen Farbstoffen kann eine Evaluation der Nieren- oder Leberorganfunktion vorgenommen werden.

Als validierte Leberfunktionsmarker eignen sich diese insbesondere als Parameter zur Messung von hepatisch eliminierten Substanzen, insbesondere bei Sepsis-assoziierter Leberdysfunktion. Diese Farbstoffe weisen eine wesentlich kürzere Verweildauer in den Hepatozyten auf. Dadurch besteht hier ein engerer Zusammenhang zwischen den PDR Messergebnissen und der tatsächlichen Exkretion in die Galle, wodurch eine bessere Aussagekraft hinsichtlich der Leberfunktion als bei der ICG- PDR besteht.

Die nierengängigen Farbstoffe können hingegen zur frühzeitigen Erfassung einer Nierendysfunktion aufgrund einer verminderten Farbstoff-Clearance eingesetzt werden.

Besonders bevorzugt ist hierbei der Farbstoff DY647, der durch seine hydrophilen Eigenschaften und die ausschließliche Exkretion über die Niere für die Nierenfunktionsdiagnostik verwendet werden kann. Die Messung der PDR dieses Farbstoffes offenbart eine einfache, schnelle und fehlerunanfällige Alternative zur Bestimmung der Creatinin-Clearance. Auch bevorzugt ist DY652.

Ebenfalls kann ICG durch hydrophile Substituenten so modifiziert werden, z.B. durch den Einbau von ionisierbaren Gruppen, dass es für die beschriebene Nierenfunktionsdiagnostik geeignet ist.

Besonders vorteilhaft ist der gleichzeitige Einsatz von mehreren Farbstoffen. So umfasst die Erfindung auch die Kombinationen eines Nierenfunktionsmarkers mit einem Leberfunktionsmarker.

Bevorzugt umfassen diese Kombinationen DY635, DY652 und/oder DY647, besonders bevorzugt ist hierbei eine Mischung aus zwei Farbstoffen von DY635, DY652 und/oder DY647, wie z.B. DY635 mit DY652 oder DY635 mit DY647.

Noch vorteilhafter ist die Kombination von kürzerwellig anregbaren Farbstoffen mit längerwellig anregbaren Farbstoffen, weil diese dann jeweils in unterschiedlichen Spektralbereichen emittieren und die Fluoreszenz der Marker somit getrennt in verschiedenen Detektionskanälen gemessen werden kann. Kombinationen von DY652 mit ICG, oder DY635 mit ICG oder DY548 mit DY635 sind dabei bevorzugt.

Damit lässt sich gleichzeitig die Nierenfunktion und die Leberfunktion mit einer einzigen Fluoreszenzmessung, so zum Beispiel über die PDR, bestimmen.

Weiterhin offenbart ist eine Vorrichtung zur Datenerfassung mit einem Marker für die Bestimmung einer Leberfunktion oder Nierenfunktion durch Fluoreszenzmessung von einem oder mehreren Polymethinfluoreszenzfarbstoffen mit einem Fluoreszenzemissionsmaximum gemessen in Ethanol von kleiner oder gleich 800 nm.

Bevorzugt umfasst die Vorrichtung wahlweise noch eines oder mehrere der folgenden Geräte:
eine Verabreichungsvorrichtung, eine Strahlungsquelle (vorzugsweise eine Laserdiode), eine Detektionsvorrichtung (vorzugsweise eine CCD Kamera) eine Datenerfassungsvorrichtung (vorzugsweise ein Computer), eine Datenspeicherungsvorrichtung (vorzugsweise ein Computer), eine Datenausgabevorrichtung (vorzugsweise ein Display, ein Ausdruck oder ein Speichermedium).

Die Erfindung befasst sich auch mit Verwendungen, oder einem Verfahren, wie oben beschrieben, zur Überwachung einer Organfunktion nach einer Leber- oder Nierentransplantation, zur Diagnostik von kritisch kranken Patienten bei einer Sepsis, bei multiplem Organversagen oder zur Vorsorgeuntersuchung.

Schließlich werden hierin nach der Erfindung noch eine Blutplasma-, Galle- oder Urinprobe zur Datenerfassung für die Bestimmung einer Organfunktion umfassend eines oder mehrerer Polymethinfluoreszenzfarbstoffe mit einem Fluoreszenzemissionsmaximum, gemessen in Ethanol, von kleiner oder gleich 820 nm als Marker beschrieben.

Bevorzugt umfasst die obige Blutserum-, Galleoder Urinprobe einen der Farbstoffe nach der Erfindung, wie hierin bereits beschrieben.

### Beispiele und Experimente

Männliche WISTAR Ratten (12-16 Wochen alt, Körpermasse 400 ± 50 g; Harlan Laboratories, Horst, Niederlande) wurden in dieser Studie verwendet. Alle Untersuchungen wurden entsprechend der deutschen Gesetze zum Schutz von Tieren und mit der Zustimmung der regionalen Tierschutzkommission in Bad Langensalza, Thüringen, Deutschland vorgenommen. Während der chirurgischen Präparation und der experimentellen Untersuchungen wurde die Körpertemperatur mit jeweils einer Wärmeplatte und einem heizbaren Objekttisch des Mikroskops auf 37° konstant gehalten. Alle Präparationen wurden unter aseptischen Bedingungen vorgenommen.

Es wurde ein spezielles Modell einer polymikrobiellen Sepsis mit einigen Modifikationen eingesetzt, welche ursprünglich durch Bauhofer beschrieben wurde (Bauhofer et al., 2004, 2006; Lorenz et al., 2004). Bei diesem Modell wird die Sepsis durch peritoneale Kontamination und Infektion (PCI) induziert.

Für die PCI wurde ein standardisiertes menschliches Stuhlinokulum eingesetzt. Unmittelbar nach der Einsammlung wurde die Stuhlprobe mit pre-reduziertem Thioglycolate-Bouillon, enthaltend 10-% steriles Bariumsulfat und 10% Glycerin, 1 : 1 verdünnt und anschließend unter anaeroben Bedingungen homogenisiert. Schließlich wurden die Stuhlproben bei -80 ° C bis zur Verwendung gelagert. Vor der Inokulation wurden die Stuhlproben 1:4 in 0,9% NaCl verdünnt. Das Inokulum wurde mikrobiologisch analysiert, um die Anzahl der Stämme nach der Lagerung zu ermitteln. Die Ratten wurden 24 h vor der PCI instrumentiert und mit Desflurane (Baxter, München, Deutschland) über eine Gesichtsmaske anästhesiert. Für den Flüssigkeitsersatz wurde ein Jugularvenenkatheter (Polyethylene, autoklavensterilisiert, OD:1,0 mm, Portex, UK) eingesetzt und subkutan bis zum Genickansatz getunnelt. Der Katheter war auf einem Drehgelenkshaltesystem montiert, welches eine uneingeschränkte Bewegung in dem Käfig nach der Erholung von der Anästhesierung ermöglichte.

In einleitenden Studien wurde die Dosis des Inokuklums so angepasst, dass eine 100-%ige Mortalitätsrate innerhalb von 40 h erzielt wurde. Die Tiere wurden zufällig in die Kontollgruppe (sham) oder in die PCI Gruppe eingeordnet. Das Inokulum wurde, unter kurzer Äthernarkose, mit einer 21G Kanüle in die Beckenregion intraperitoneal injiziert. Die Tiere der Kontrolle (sham) erhielten 0,9 % NaCl in der gleichen Menge. Nach der Prozedur erhielten beide Gruppen Flüssigkeitsersatz {10ml/kg/KG/h, Jonosteril®, Fresenius Kabi GmbH, Deutschland) sowie Futter und Wasser ad libitum.

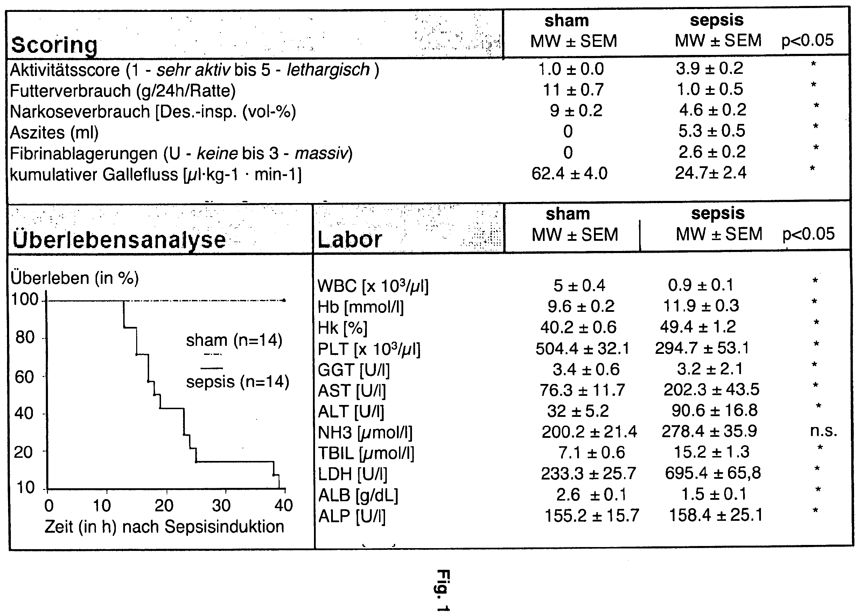

Um den klinischen Status bei einem zuvor bestimmten Zeitpunkt für zukünftige Experimente bestimmen zu können, wurde die Aktivität entsprechend früheren Beschreibungen mit einem Punktesystem quantifiziert (Van Griensven et al., 2002). Die erzielte Punktezahl reflektiert die spontane Aktivität sowie die Reaktion auf exogene Stimulationen. Die Punktezahl reichte von 1 bis 5, wobei 1 eine hohe Aktivität repräsentiert und die Aktivität stetig bis 5 abnimmt, welches schließlich für einen lethargischen Zustand steht. Zudem wurde die Nahrungsaufnahme (g/Ratte) bei 15 h gemessen. Nach der Laparotomie wurde das peritoneale Flüssigkeitsvolumen (ml/Ratte) gemessen und die Erzeugung eines Fibrinfilms anhand einer Punktebewertung zwischen 0 (keine) bis 3 (massiv) quantifiziert (siehe hierzu auch Figur 1). Die Punkteverteilung wurde unabhängig voneinander in einer Blindbewertung durch zwei der Autoren durchgeführt. Beide Beobachter bewerteten jeweils jede Ratte. Die Punktezuordnung von jeder einzelnen Ratte wurde letztlich aus dem Durchschnitt von beiden Werten gebildet.

### In vivo Beurteilung der hepatischen Clearance-Funktion

Die Plasma Abnahmerate, genannt Plasma Disappearance Rate (PDR), sowie die Gallenausscheidung der Farbstoffe DY635 (Dyomics, Jena, Deutschland) und Indozyaningrün als Vergleichsexperiment (ICG; Pulsion, München, Deutschland) wurden in vivo bestimmt.

Die Tiere wurden 15 h nach der Sepsis bzw. Induktion der Kontrollgruppe anästhesiert. Zumal sich herausgestellt hat, dass Ketamin den bewussten Zustand, gemessen durch Herzleistung und regionalem Blutfluss, am nächsten kommend darstellt (Seyde et al. 1984), wurden die Ratten mit einer Mischung von Ketamin (Ketamin® 50mg/ml; Deltaselect, Pfullingen, Deutschland) und Midazolam (Midazolam-ratiopharm® 5mg/m!; Ratiopharm, Ulm, Deutschland) anästhesiert, welches über einen Jugularvenenkatheter (Polyethylene, über einem Autoklaven sterilisiert, OD: 1,0 mm, Portex, UK) mit einer Dosis von 40 mg/kg bzw. 4 mg/kg Körpergewicht verabreicht wurde.

Ein arterieller Katheter wurde zur hämodynamischen Überwachung eingesetzt. Eine Mittellinien und rechte su bcostale Laparotomie wurde vorgenommen und der Ductus Choledochus wurde mit einem Polyethylenkatheter (Polyethylene, autoklavensterilisiert, OD: 1,0 mm, Portex, UK) für die Gallensammlung mit einer Kanüle versehen. Schließlich wurde die Wunde mit Mersüene 2-0 (Ethicon, Hamburg, Deutschland) verschlossen und die Anästhesierung aufrechterhalten.

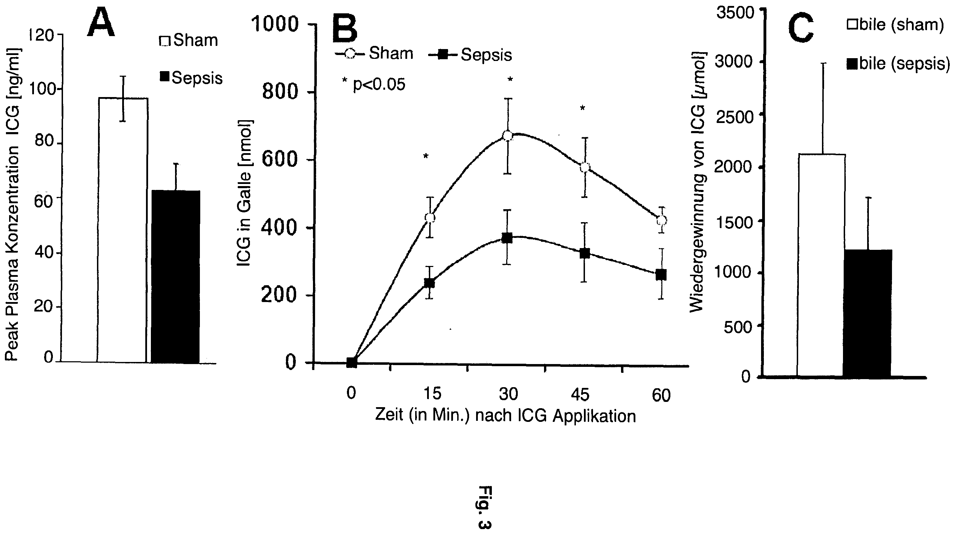

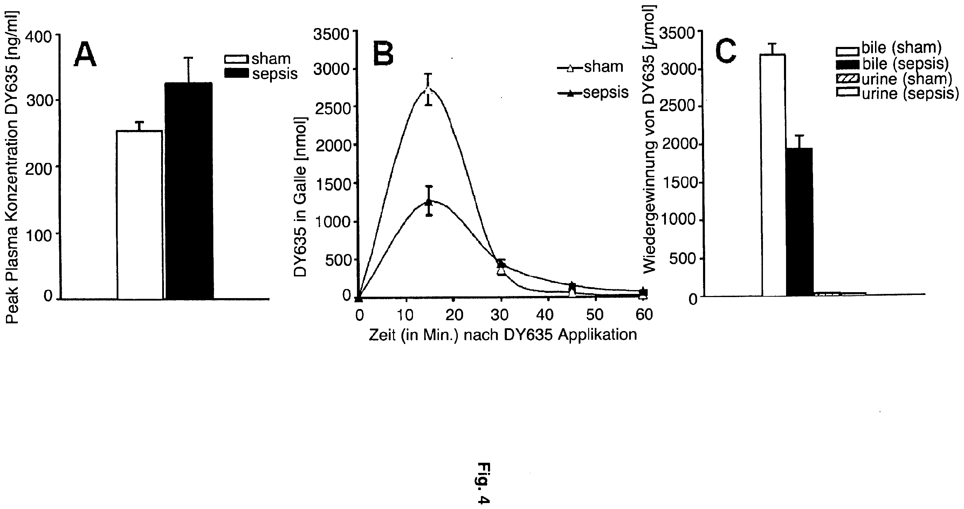

Der Farbstoff wurde über einem Jugularvenenkatheter verabreicht. Die Blutproben und Gallenproben wurden zum Nullpunkt und anschließend alle 15 min für eine Zeitdauer von 60 min abgenommen. Blutverluste durch diese Blutproben wurden durch Infusionen adäquat ausgeglichen. Die hämodynamische Überwachung wurde durchgängig ausgeführt.
Die Ergebnisse werden in den Figuren 3 und 4 dargestellt.

### In vivo Bestimmung des hepatozellulären und canaiiculären Transports

Veränderungen in der hepatozellulären Aufnahme und der canaiiculären Eliminierung wurden 15 h nach der Sepsis bzw. Induktion der Kontrollgruppe durch in vivo Imaging des ausschließlich hepatozytisch eliminierten Farbstoffs Indozyaningrün (Pulsion, München, Deutschland) und von DY 635 mit einem invertierten Epifluoreszenzmikroskop (AxioObserver® Z1 ; Zeiss, Jena, Deutschland), ausgestattet mit ei nem computergesteuerten hochpräzisen Rastertisch (Märzhäuser, Wetzlar, Deutschland), gemessen.

Unter Verwendung eines x40/0.6 Objektivs wurde die Leberoberfläche in Epi-Anordnung mit einer 100 W Quecksilberlampe bestrahlt und unter Verwendung von zwei Filtersätzen mit einem 365 nm bis 395 nm Anregungs- und 445 nm bis 450 nm Emissionsbandpassfilter die Leberarchitektur durch natürliche Autofluoreszenz der hepatozellulär reduzierte Pyridin Nukleotide (NADH) visualisiert. Zum Abbilden des Farbstoffs Indozyaningrün wurden 775 nm bis 805 nm Anregungs- und 845 nm bis 855 nm Emissionsbandpassfilter eingesetzt.

Alle Mikroskopabbildungen wurden auf eine hochauflösende CCD Digital Videokamera (C9100-13; Hammamatsu Photonics, Japan) abgebildet, welches mit einem PC mit Microsoft-Windows® basierter Software für die Bedienung des Mikroskops, die Navigation der Rasterbühne und die Bildanalyse (AxioVision® Rel. 4.6; Zeiss, Jena, Deutschland) ausgestattet war.

Die Tiere wurden mit Ketamin und Midazolam wie oben 15 h nach der Sepsis bzw. Induktion der Kontrollgruppe anästhesiert. Eine Mittellinien- und linksseitige subcostale Laparotomie wurde zur Definition des linken Leberlappens vorgenommen. Um respiratorische Bewegungen zu minimieren, wurden hepatische Ligamente getrennt. Der Darm wurde mit Saline-getränktem Verbandmull abgedeckt, um eine Gewebedehydration zu verhindern. Nachdem die Tiere auf die Mikroskopbühne übertraaen worden sind, wurden die Ratten auf ihre linke Seite aedreht. Der mobilisierte linke Leberlappen wurde freigelegt und die pragmatische Oberfläche auf einem Mikroskopdeckglass (#1; Menzel, Braunschweig, Deutschland) positioniert und leicht mit Histoacryl (Braun, Melsungen, Deutschland) fixiert. Typischerweise konnte somit eine Fläche von 1 -1.5 cm² des Leberlappens mit Intravitalmikroskopie untersucht werden. Um die Austrocknung während der Intravitalmikroskopie zu verhindern, wurde die Leber regelmäßig mit warmer Ringer-Lösung (37°) überspült.
In einem ersten Schritt wurde die hepatozelluläre NADH Autofluoreszenz untersucht, um die mitochondriale Oxidationsstufe zu ermitteln (Vollmar et al. 1997). Unter Verwendung eines x5 Objektivs und einem 365 nm bis 395 nm Anregungs- und 445 nm bis 450 nm Emissionsbandpassfilter wurden 5 zufällig gewählte Sichtfelder pro Tier aufgenommen. Für alle Sichtfelder wurden Standardeinstellungen für die Verstärkung, Belichtungszeit und Bildkontrastverstärkung verwendet. Die NADH Autofluoreszenz wurde densitometrisch bestimmt und als Durchschnittsintensität pro Sichtfeld (arbitrary units [aU]) ausgedrückt.

Für die Bestimmung der hepatischen Mikrozirkulation wurden mit einem x20 Objektiv, aber unter Verwendung des gleichen Filterblocks, fünf 15 s Videosequenzen aufgenommen.

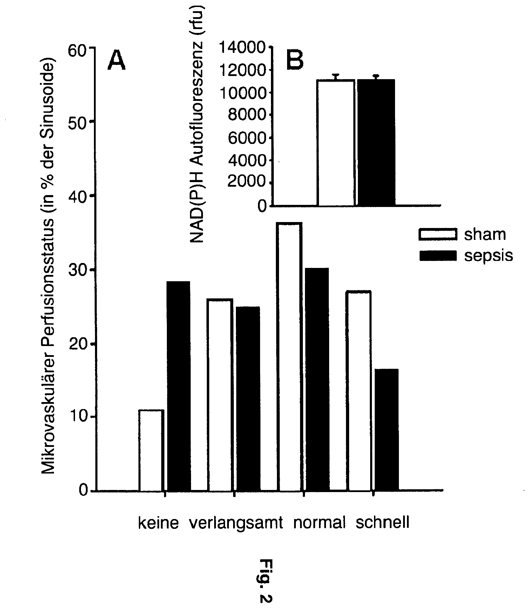

Der Zusammenbruch der sinusoidalen Perfusion wurde anhand der Anzahl der nicht perfundierten hepatischen Sinusoiden bestimmt (gemessen in Prozent der Sinusoiden die einen 100 µm Kreis schneiden). Um auch Informationen über die Art des Durchflusses in den perfundierten Sinusoiden zu sammeln, wurde der mikrozirkulatorische Fließindex (DeBacker et al. 2007) mit einigen Modifikationen bestimmt (siehe Figur 2). Dabei wurde eine Videosequenzen in vier Quadranten unterteilt und die vorherrschende Fließart (1=langsam, 2=normal, 3=zügig) in jedem Quadranten durch zwei unabhängige Beobachter bestimmt.

Um 16 Regionen (regions of interest: ROI) für die in vivo Abbildungen des Farbstoffes aufzufinden, wurde die Leberoberfläche gerastert; 8 perizentrale and 8 periportale Regionen wurden ermittelt und deren exakte Position auf der computergesteuerten Rasterbühne (scanning stage) mit der AxioVision® Software bestimmt und gespeichert, so dass die pre-definierten ROI während der zukünftige Experimente zugänglich waren. Die Fototoxizität wurde dadurch begrenzt, dass während der Identifikation der ROI, die Lichttransmission mit einem Neutraldichtefilter auf 20 % reduziert worden ist.

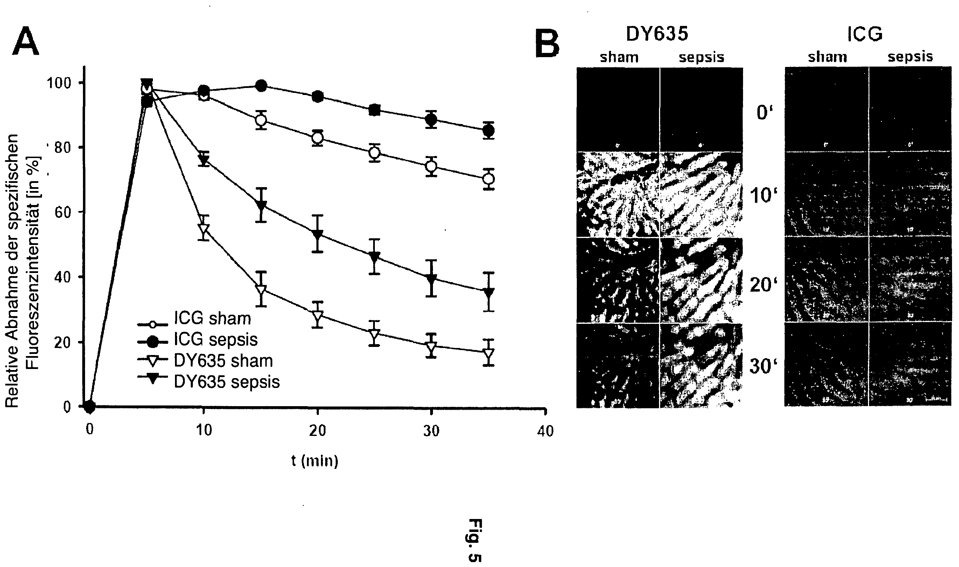

Vor der Zugabe des Indozyaningrüns, bzw. DY 635 wurden Basislinien-Abbildungen der definierten ROI mit dem AxioVision® Multikanalmodul (x40/0.6 Objektiv, 365 nm bis 395 nm Anregungs- und 445 nm bis 450 nm Emissionsbandpassfilter und 775 nm bis 805 nm Anregungs- und 845 nm bis 855 nm Emissionsbandpassfilter) aufgenommen. Die optimalen Einstellungen für die Verstärkung, Belichtungszeit und Bildkontrastverstärkung wurden in Vorversuchen ermittelt. Danach wurde der Farbstoff über ein Jugularvenenkatheter verabreicht und Abbildungen von sämtlichen ROI alle 5 min über eine Zeitperiode von 35 min aufgenommen (siehe Figur 5).

Dis Fiuoreszsnzintensiiät von Indozyaningrün, bzw. DY 635, weiches im mikrozirkulatorischen und hepatischen Gewebe verteilt vorlag, wurde densitometrisch quantifiziert und als durchschnittliche Intensität pro ROI ausgedrückt (arbitrary units [aU]).

### In vivo Bestimmung der Nierenfunktion

Weitere Experimente wurden zur Erfassung der Nierenfunktion mit DY652 (Daten hier nicht gezeigt) und DY647 durchgeführt.

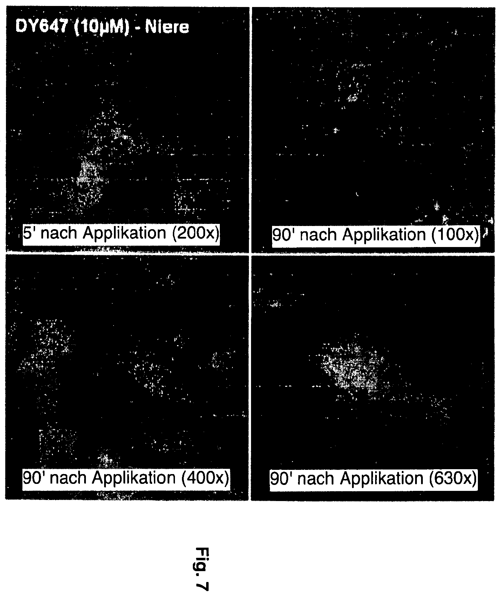

Die Ergebnisse für DY 647 werden in der Figur 7 gezeigt. In den Aufnahmen zu sehen sind das Nierengewebe und das weitverzweigte Nierengefäßsystem. Wie auf den Aufnahmen zu erkennen ist, hat der Farbstoff das Gefäßsystem verlassen und ist im Nierengewebe - hier in den Nierentubuli - zu sehen. Nach Applikation wurde der Farbstoff im Urin nachgewiesen.

### In vivo Quantifizierung der Organdysfunktion während Sepsis

Vor der chirurgischen Präparation für die Intravitalmikroskopie wurden Blutproben zur Bestimmung der Organdysfunktion unter Narkose aus dem refrobu/baren Plexus der Tiere entnommen.

Anschließend wurden mittels automatisierter veterinärer Hämatologie und klinisch chemischer Analysatoren die folgenden Werte bestimmt:
Hämoglobin (Hb), Hämatokrit (Hk), Anzahl der weißen Blutkörperchen (white blood cell count; WBC), Anzahl der Blutplättchen (Platelet count PLT), Aspartate Aminotransferase (AST), γ-Glutamyltransferase (γGT), Bilirubin, Albumin, Ammoniak (NH₃) und Laktat Dehydrogenase (LDH) (Fuji Dri-Chem 3500i und Poch-100iv-Diff, Sysmex, Leipzig, Deutschland).
Diese Daten werden in Figur 1 zusammengefasst.

Die Erfindung wird nun anhand der folgenden Figuren und Ergebnisse näher erläutert.
Figur 1 : Charakterisierung des verwendeten Sepsismodells. Gezeigt ist die Überlebensanalyse nach Sepsisinduktion. Die Intervention erfolgte 15 Stunden nach Sepsisinduktion- zu diesem Zeitpunkt betrug die Mortalität ca. 20%. Vor der Untersuchung wurden die Tiere klinisch beurteilt und unterschiedliche Laborparameter zur Beurteilung von Organdysfunktionen bestimmt.
Figur 2: Evaluation von Mikrozirkulation und Energiestatus 15 Stunden nach Sepsisinduktion. Der Energiestatus war nicht kompromittiert, die Mikrozirkulation war moderat eingeschränkt.
Figur 3: ICG Kinetik in Plasma und Galle. (A) zeigt die Plasmakonzentration von ICG unmittelbar nach Applikation. (B) gibt den zeitlichen Verlauf der ICG-Konzentration in der Galle wider. (C) zeigt die kumulative Stoffmenge an ICG an, die in einem Zeitraum von 60 Minuten nach Applikation in der Galle gesammelt wurde.
Figur 4: DY635 Kinetik in Plasma und Galle. (A) zeigt die Plasmakonzentration von DY635 unmittelbar nach Applikation. (B) gibt den zeitlichen Verlauf der DY635- Konzentration in der Galle wider. (C) zeigt die kumulative Stoffmenge an DY635 an, die in einem Zeitraum von 60 Minuten nach Applikation in der Galle gesammelt wurde.
Figur 5: In vivo Imaging von DY635 und ICG in der Leber. (A) zeigt den relativen Abfall der Fluoreszenzintensität beider Farbstoffe nach Applikation. In den septischen Tieren resultiert eine Akkumulation des Farbstoffes (A,B).
Figur 6: Vergleich der Zytotoxizität zwischen ICG und DY635. Es ist jeweils das Überleben kultivierter Hepatozyten 24 h nach Zugabe unterschiedlicher Konzentrationen der Farbstoffe gezeigt (MTT Assay).
Figur 7: In vivo Imaging der Exkretion von DY647 über die Niere. 5 Minuten nach Applikation ist der Farbstoff im Gefäßsystem der Niere zu sehen. Nach 90 Minuten hat der Farbstoff das Gefäßsystem verlassen und ist in den Nierentubuli zu sehen.

Es wurden Vergleichsstudien anhand des bekannten Leberfunktionsmarkers ICG vorgenommen.

Figur 1 charakterisiert das zugrunde liegende Sepsismodell. Insgesamt litten die Tiere zum Untersuchungszeitpunkt an einer schweren Sepsis. Ein Fünftel der Tiere war bereits verstorben und die noch lebenden Tiere wiesen deutliche Krankheitszeichen auf. Die Laborchemie weist zudem auf eine schwere hepatische Dysfunktion hin.

Mikrozirkulation und Energiestatus wurden ebenfalls untersucht, da diese - neben den zellulären Transportsystemen - den hepatozellulären Transport determinieren. Figur 2 zeigt, dass der Energiestatus in septischen Tieren nicht beeinträchtigt war. Hinsichtlich der Mikrozirkulation ließ sich in den septischen Tieren eine nur geringfügige Kompromittierung feststellen. In den septischen Tieren waren insgesamt weniger Sinsuoide perfundiert. Funktionell hatte dies jedoch keine Bedeutung, da 15 Minuten nach Applikation beider Farbstoffe, nahezu kein Farbstoff im Plasma mehr nachweisbar war (Daten sind nicht gezeigt).

Figur 3 und 4 zeigen jeweils Aspekte der Kinetik der beiden Farbstoffe in Serum und Galle. Unmittelbar nach Applikation ist weniger ICG im Plasma der septischen Tiere nachweisbar. Die Aufnahme ist somit nicht verzögert. Die ebenfalls dargestellte Messung von ICG in der Galle zeigt einen Initialanstieg der Konzentration, der ab 30 min langsam zurückgeht. Hierbei zeigt sich, dass die Kontrollgruppe sowohl einen stärkeren Anstieg, als auch eine schnellere Abnahme der ICG Konzentration aufweist. Dies lässt sich durch eine höhere aktive Exkretion über die kanalikuläre Membran in die Galle bei der Kontrollgruppe erklären. Nach 60 Minuten waren noch immer hohe ICG Werte messbar. Dies ist in Figur 3B an der durchgängigen Linie erkennbar, die die Konzentration in der Galle darstellt. Die gestörte exkretorische Organfunktion in der Sepsisgruppe ist im Vergleich zu der Kontrollgruppe (sham) eingeschränkt und dieser Unterschied lässt sich anhand der ICG Farbstoffmessungen in der Galle ermitteln. Daher bestätigen diese Ergebnisse die Eignung von ICG als Leberfunktionsmarkernicht jedoch im klassischen Sinne einer Messung der Plasmaverschwinderate (PDR-ICG). ICG wurde in dem hier aufgezeigten Setting als Marker für die exkretorische Leberfunktion eingesetzt. Zahlreiche wissenschaftliche Arbeiten weisen daraufhin, dass vor allem die canaliculären Transportsysteme des Hepatozyten sehr sensitiv gegenüber einem pro-infiammatorischen Zustand sind und dadurch frühzeitig kompromittiert werden. Herkömmliche PDR-ICG Messungen könnten daher "falsch-hohe" PDR's anzeigen, da nicht unbedingt ein direkter Zusammenhang zwischen gemessener Serumfluoreszenzabnahme und exkretorischer Leberfunktion besteht. Die PDR-ICG misst lediglich die Aufnahme und Einlagerung des Farbstoffs in den Hepatozyten und kann keine Aussage bezüglich der tatsächlichen Exkretionsleistung der Leber machen.

Die Untersuchungen zur ICG Kinetik in Plasma und Galle wurden analog für DY635 durchgeführt (Figur 4). Unmittelbar nach Applikation von DY635 war in den gesunden Tieren weniger DY635 im Plasma nachweisbar als in den kranken Tieren. Die hepatozelluläre Aufnahme erfolgte also prolongiert. Allerdings wurde sowohl in der Kontrollgruppe, als auch in der Sepsisgruppe der Farbstoff innerhalb von 15 min komplett in die Hepatozyten aufgenommen (Daten sind nicht gezeigt). Nach 60 min konnte der Farbstoff zudem weder in der Niere, noch im Urin festgestellt werden. Die Messung von DY635 in der Galle zeigt, dass die Kontrollgruppe sowohl einen stärkeren Anstieg, als auch eine schnellere Abnahme der DY635 Konzentration aufweist als die Sepsisgruppe. Daher bestätigen diese Ergebnisse die Eignung von DY635 als Leberfunktionsmarker.

Hinzu kommt allerdings, dass der Anstieg und die darauf folgende Abnahme auch ausgeprägter sind als bei den Vergleichsversuchen für ICG. Der Farbstoff DY635 wird also schneller canaliculär in die Gallenflüssigkeit ausgeschieden.

Zudem ist der Unterschied in der Gallenexkretion zwischen der Kontrollgruppe und der Sepsisgruppe bei DY635 deutlich ausgeprägter als für ICG. Nach 60 min waren in den Kontrolltieren bereits 60% des applizierten DY635 canaliculär ausgeschieden. Hingegen waren im gleichen Zeitraum in den septischen Tieren nur 34% ausgeschieden. Der Farbstoff DY635 akkumuliert bereits in gesunden Tieren deutlich weniger als ICG, wobei bei eingeschränkter exkretorischer Leistung ebenfalls eine Akkumulation wie bei ICG resultiert.

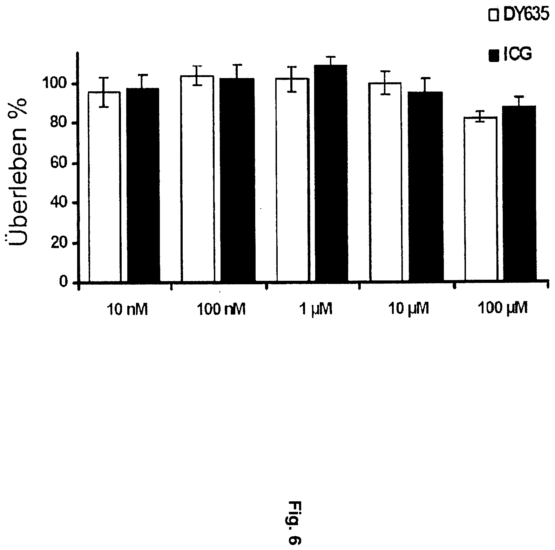

Figur 6 zeigt, dass es im Zytotox-Assay im Vergleich zu ICG keinen Hinweis auf eine erhöhte Toxizität gibt.

Figur 5 zeigt die Ergebnisse des in vivo imagings für ICG und DY635. Mittels Intravitalmikroskopie wurde die Fluoreszenz von DY635 bzw. ICG in periportalen und perivenösen Leberarealen in Zeitintervallen nach Applikation erfasst. Die Fluoreszenz gibt vor allem Auskunft über die hepatozelluläre Akkumulation der Farbstoffe. In Ubereinstimmung mit den Konzentrationsbestimmungen beider Farbstoffe in der Galle nimmt die spezifische Fluoreszenzintensität und damit die Farbstoffkonzentration im Organgewebe bei DY635 deutlich stärker ab als für ICG. Auch die Differenz zwischen Sepsisgruppe und Kontrollgruppe ist insbesondere in den ersten Minuten nach der Verabreichung stärker ausgeprägt als bei ICG. Die korrespondierenden Bilder zeigen eine deutlichere Auflösung von DY635. Insbesondere die se Tatsache, als auch der Fakt, dass DY635 nicht bereits in gesunden Tieren zur Akkumulation neigt, machen DY635 besonders für in vivo imaging Anwendungen geeignet.

Im Rahmen von in vivo Messungen an der Niere zeigte sich, dass der wasserlösliche Farbstoff DY647 selektiv über die Niere ausgeschieden wird (Figur 7). Eine Ausscheidung bei einer zugrunde Hegenden Dysfunktion führt zu einer verzögerten Ausscheidung und somit zu einer längeren Nachweisbarkeit im Blut. Analog verhält es sich mit DY652. Damit eignen sich die nierengängige Farbstoffe DY652 und DY647 als Nierenfunktionsmarker in der Organdiagnostik.

## Patentansprüche

1. Ein oder mehrere Polymethinfluoreszenzfarbstoffe zur Verwendung als Marker zur Datenerfassung für die Bestimmung einer Organfunktion *in vivo*, wobei der eine oder die mehreren Polymethinfluoreszenzfarbstoffe ein Fluoreszenzemissionsmaximum, gemessen in Ethanol, von kleiner oder gleich 820 nm aufweist, und wobei das Organ, dessen Funktion bestimmt wird, die Leber und/oder die Niere ist.

2. Ein oder mehrere Polymethinfluoreszenzfarbstoffe mit einem Fluoreszenzemissionsmaximum, gemessen in Ethanol, von kleiner oder gleich 820 nm zur Verwendung in einem Verfahren zur Datenerfassung für die Bestimmung einer Organfunktion umfassend die Schritte:
a) Radiative Anregung *in vivo* von dem einem oder den mehreren Polymethinfluoreszenzfarbstoffen mit einem Fluoreszenzemissionsmaximum, gemessen in Ethanol, von kleiner oder gleich 820 nm, als Marker im Blut, in einem Gewebe oder Organ und/oder in der Galle oder Urin, mit einer Strahlungsquelle,
b) Detektion der Fluoreszenzemission des einen oder der mehreren Polymethinfluoreszenzfarbstoffe mit einer Detektionsvorrichtung,
c) Erfassung der Daten der gemessenen Fluoreszenzintensität mit einer Datenerfassungsvorrichtung,
wobei das Organ, dessen Funktion bestimmt wird, die Leber und/oder die Niere ist.

3. Ein oder mehrere Polymethinfarbstoffe zur Verwendung nach Anspruch 1 oder 2, wobei der Farbstoff ein Benzopyrylium-basiertes Hemicyanin oder ein Indodicarbocyanin-Chromophor ist.

4. Ein oder mehrere Polymethinfarbstoffe zur Verwendung nach einem der Ansprüche 1 bis 3, wobei der eine oder die mehreren Farbstoffe ein unsymmetrisches Polymethin, aufweisend einer substituierten omega-(Benz[b]pyran-4-yliden)alk-1-enyl)-Einheit der allgemeinen Struktur I ist 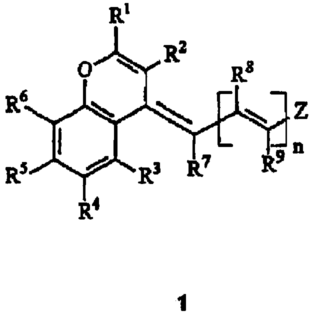 mit Z als substituierten Benzooxazol-, Benzothiazol-, 2,3,3-Trimethylindolenin-, 2,3,3-Trimethyl-4,5-benzo-3H-indolenin-, 2- und 4-Picolin-, Lepidin-, Chinaldin- sowie 9-Methylacridinderivaten der allgemeinen Formeln IIa oder IIb oder IIc 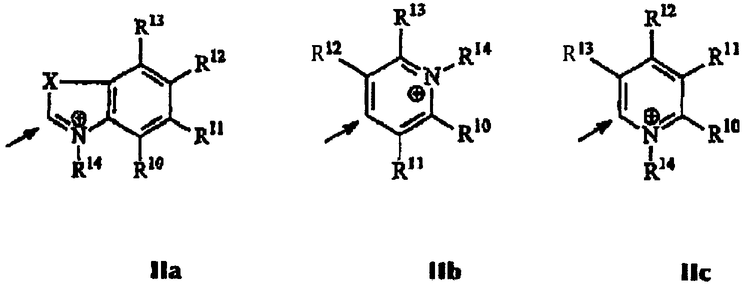 wobei
a. X für ein Element aus der Gruppe O, S, Se oder das Strukturelement N-alkyl oder C(alkyl)₂ steht,
b. n für die Zahlenwerte 1, 2 oder 3 steht,
c. R¹ - R¹⁴ gleich oder unterschiedlich sind und Wasserstoff, ein oder mehrere Alkyl, oder Aryl-, Heteroaryl- oder heterocycloaliphatische Reste, eine Hydroxy- oder Alkoxygruppe, eine alkylsubsituierte oder cyclische Aminfunktion sein können und/oder zwei orthoständige Reste, z.B. R¹⁰ und R¹¹, R⁴ und R⁵ und/oder R⁶ und R⁵ zusammen einen weiteren aromatischen Ring bilden können,
d. mindestens einer der Substituenten R¹ - R¹⁴ einen solubilisierenden bzw. ionisierbaren bzw. ionisierten Substituenten, wie Cyclodextrin, Zucker, SO₃⁻, PO₃⁻, COO⁻, oder NR₃⁺ darstellen kann, der die hydrophilen Eigenschaften dieser Farbstoffe bestimmt, wobei dieser Substituent auch über eine Spacergruppe am Farbstoff angebunden sein kann,
und
R¹ einen Substituenten darstellt, der in alpha-Position zum Pyran-Ring ein quartäres C-Atom aufweist, bevorzugt sind t-Butyl und Adamantyl.

5. Ein oder mehrere Polymethinfarbstoffe zur Verwendung nach einem der Anprüche 1 bis 4 für die Nierenfunktionsbestimmung, wobei der eine oder die mehreren Farbstoffe in Wasser löslich sind, aber in DMF oder DMSO unlöslich sind
oder
für die Leberfunktionsbestimmung, wobei der eine oder die mehreren Farbstoffe in DMF oder DMSO löslich und in Wasser unlöslich sind.

6. Ein oder mehrere Polymethinfarbstoffe zur Verwendung nach einem der Ansprüche 1 bis 5, wobei der eine oder die mehreren Farbstoffe in physiologischer Lösung eine neutrale Ladung aufweisen oder höchstens einfach geladen vorliegen und/oder bevorzugt höchstens zwei solubilisierende bzw. ionisierbare bzw. ionisierte Substituenten aufweisen
oder
wobei der eine oder die mehreren Farbstoffe in physiologischer Lösung mindestens zweifach geladen vorliegen und/oder bevorzugt mindestens drei oder vier solubilisierende bzw. ionisierbare bzw. ionisierte Substituenten aufweisen.

7. Ein oder mehrere Polymethinfarbstoffe zur Verwendung nach einem der Ansprüche 1 bis 6, wobei der eine oder die mehreren Farbstoffe ausgewählt sind aus der Gruppe umfassend: DY630, DY631, DY632, DY633, DY634, DY635, DY636, DY647, DY648, DY649, DY650, DY651 und DY652 sowie DY590, DY548, DY495, DY405.

8. Ein oder mehrere Polymethinfarbstoffe zur Verwendung nach einem der Ansprüche 1 bis 7 zur Überwachung einer Organfunktion nach einer Leber oder Nierentransplantation, zur Diagnostik von kritisch kranken Patienten bei einer Sepsis oder bei multiplem Organversagen oder zur Vorsorgeuntersuchung.

9. Verfahren zur Datenerfassung für die Bestimmung einer Organfunktion *in vitro* / *ex vivo* umfassend die Schritte:
a) Radiative Anregung von einem oder mehreren Polymethinfluoreszenzfarbstoffen mit einem Fluoreszenzemissionsmaximum, gemessen in Ethanol, von kleiner oder gleich 820 nm, als Marker im Blut, in einem Gewebe oder Organ und/oder in der Galle oder Urin, mit einer Strahlungsquelle,
b) Detektion der Fluoreszenzemission des einen oder der mehreren Polymethinfluoreszenzfarbstoffe mit einer Detektionsvorrichtung,
c) Erfassung der Daten der gemessenen Fluoreszenzintensität mit einer Datenerfassungsvorrichtung,
wobei das Organ, dessen Funktion bestimmt wird, die Leber und/oder die Niere ist.

10. Verfahren nach Anspruch 9, wobei der Farbstoff ein Benzopyrylium-basiertes Hemicyanin oder ein Indodicarbocyanin-Chromophor ist.

11. Verfahren nach Anspruch 9 oder 10, wobei der eine oder die mehreren Farbstoffe ein unsymmetrisches Polymethin, aufweisend einer substituierten omega-(Benz[b]pyran-4-yliden)alk-1-enyl)-Einheit der allgemeinen Struktur I ist 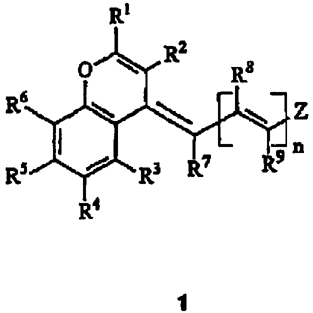 mit Z als substituierten Benzooxazol-, Benzothiazol-, 2,3,3-Trimethylindolenin-, 2,3,3-Trimethyl-4,5-benzo-3H-indolenin-, 2- und 4-Picolin-, Lepidin-, Chinaldin- sowie 9-Methylacridinderivaten der allgemeinen Formeln IIa oder IIb oder IIc 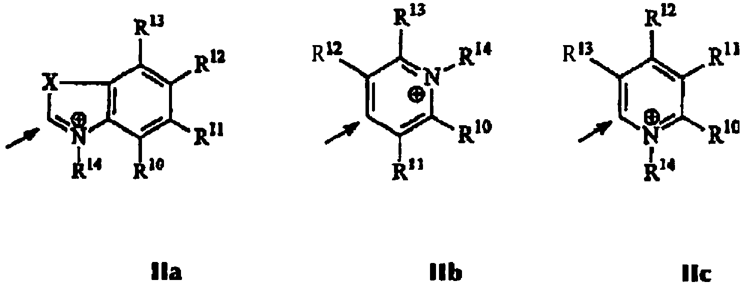 wobei
a. X für ein Element aus der Gruppe O, S, Se oder das Strukturelement N-alkyl oder C(alkyl)₂ steht,
b. n für die Zahlenwerte 1, 2 oder 3 steht,
c. R¹ - R¹⁴ gleich oder unterschiedlich sind und Wasserstoff, ein oder mehrere Alkyl, oder Aryl-, Heteroaryl- oder heterocycloaliphatische Reste, eine Hydroxy- oder Alkoxygruppe, eine alkylsubsituierte oder cyclische Aminfunktion sein können und/oder zwei orthoständige Reste, z.B. R¹⁰ und R¹¹, R⁴ und R⁵ und/oder R⁶ und R⁵ zusammen einen weiteren aromatischen Ring bilden können,
d. mindestens einer der Substituenten R¹ - R¹⁴ einen solubilisierenden bzw. ionisierbaren bzw. ionisierten Substituenten, wie Cyclodextrin, Zucker, SO₃⁻, PO₃⁻, COO⁻, oder NR₃⁺ darstellen kann, der die hydrophilen Eigenschaften dieser Farbstoffe bestimmt, wobei dieser Substituent auch über eine Spacergruppe am Farbstoff angebunden sein kann,
und
R¹ einen Substituenten darstellt, der in alpha-Position zum Pyran-Ring ein quartäres C-Atom aufweist, bevorzugt sind t-Butyl und Adamantyl.

12. Verfahren nach einem der Ansprüche 9 bis 11 für die Nierenfunktionsbestimmung, wobei der eine oder die mehreren Farbstoffe in Wasser löslich sind, aber in DMF oder DMSO unlöslich sind oder
für die Leberfunktionsbestimmung, wobei der eine oder die mehreren Farbstoffe in DMF oder DMSO löslich und in Wasser unlöslich sind.

13. Verfahren nach einem der Ansprüche 9 bis 12,
wobei der eine oder die mehreren Farbstoffe in physiologischer Lösung eine neutrale Ladung aufweisen oder höchstens einfach geladen vorliegen und/oder bevorzugt höchstens zwei solubilisierende bzw. ionisierbare bzw. ionisierte Substituenten aufweisen
oder
wobei der eine oder die mehreren Farbstoffe in physiologischer Lösung mindestens zweifach geladen vorliegen und/oder bevorzugt mindestens drei oder vier solubilisierende bzw. ionisierbare bzw. ionisierte Substituenten aufweisen.

14. Verfahren nach einem der Ansprüche 9 bis 13, wobei der eine oder die mehreren Farbstoffe ausgewählt sind aus der Gruppe umfassend:
DY630, DY631, DY632, DY633, DY634, DY635, DY636, DY647, DY648, DY649, DY650, DY651 und DY652 sowie DY590, DY548, DY495, DY405.

15. Verfahren nach einem der Ansprüche 9 bis 14 zur Überwachung einer Organfunktion nach einer Leber oder Nierentransplantation, zur Diagnostik von kritisch kranken Patienten bei einer Sepsis oder bei multiplem Organversagen oder zur Vorsorgeuntersuchung.

16. Blutserum-, Galle- oder Urinprobe zur Verwendung zur Datenerfassung für die Bestimmung einer Organfunktion umfassend einen oder mehrere Polymethinfluoreszenzfarbstoffe, nach einem der Ansprüche 1 bis 8, mit einem Fluoreszenzemissionsmaximum, gemessen in Ethanol, von kleiner oder gleich 820 nm als Marker, wobei das Organ, dessen Funktion bestimmt wird, die Leber und/oder die Niere ist.

## Claims

1. One or more polymethine fluorescence dyes for use as markers for acquiring data in order to determine an organ function *in vivo*, wherein the one or more polymethine fluorescence dyes have a fluorescence emission maximum, measured in ethanol, of less than or equal to 820 nm, and wherein the organ, of which the function is determined, is the liver and/or kidney.

2. One or more polymethine fluorescence dyes with a fluorescence emission, measured in ethanol, of less than or equal to 820 nm for use in a method for acquiring data in order to determine an organ function, comprising the steps:
a) radiative excitation *in vivo* of the one or more polymethine fluorescence dyes having a fluorescence emission maximum, measured in ethanol, of less than or equal to 820 nm as markers in blood, in a tissue or organ and/or in bile or urine, by means of a radiation source,
b) detection of the fluorescence emission of the one or more polymethine dyes by means of a detection device,
c) acquisition of the data of the measured fluorescence intensity by means of a data acquisition device,
wherein the organ, of which the function is determined, is the liver and/or kidney.

3. One or more polymethine dyes according to Claim 1 to 2, wherein the dye is a benzopyrylium-based hemicyanine or an indodicarbocyanine chromophore.

4. One or more polymethine dyes for use according to one of claims 1 to 3, wherein the one or more dyes is an asymmetrical polymethine having a substituted omega-(benz[b]pyran-4-yliden)alk-1-enyl) unit of the general structure 1: 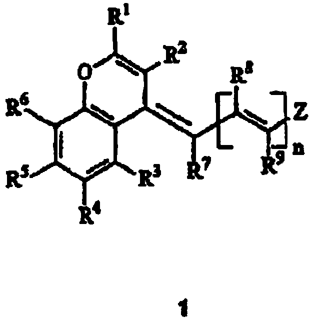 with Z as substituted benzoxazole-, benzothiazole-, 2,3,3-trimethylindolenin, 2,3.3-trimethy)-4,5-benzo-3H-indotenine-, 2- and 4-picoline-, lepidine-, quinaldine- and 9-methylacridine- derivatives of the general formulas IIa or IIb or IIc 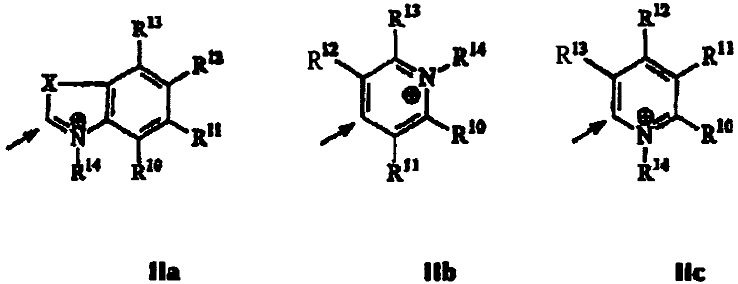 wherein
a. X stands for an element from the group O, S, Se or the structural element N-alkyl or C(alkyl)₂,
b. n stands for the numbers 1, 2 or 3,
c. R¹-R¹⁴ are the same or different and can be hydrogen, one or more alkyl, or aryl, heteroaryl, or heterocycloaliphatic residues, a hydroxyl group or alkoxy group, an alkyl-substituted or cyclic amine function, and/or two ortho residues, e.g. R¹⁰ and R¹¹, R⁴ and R⁵ and/or R⁶ and R⁵ together can form a further aromatic ring,
d. at least one of the substituents R¹-R¹⁴ can represent a solubilizing or ionisable or ionised substituent, such as cyclodextrin, sugar, SO₃⁻, PO₃⁻, COO', or NR₃⁺, which determines the hydrophilic properties of these dyes, wherein this substituent can also be bonded to the dye via a spacer group,
and
R¹ represents a substituent that has a quaternary C atom in the alpha position at the pyran ring, preferred are t-butyl or adamantyl.

5. One or more polymethine dyes for use according to one of claims 1 to 4 for determining kidney function, wherein the one or more dyes are soluble in water but are insoluble in DMF or DMSO
or
for determining liver function, wherein the one or more dyes are soluble in DMF or DMSO and are insoluble in water.

6. One or more polymethine dyes for use according to one of Claims 1 to 5, wherein the one or more dyes have a neutral charge in physiological solution or are provided at most singly charged and/or preferably have at most two solubilising or ionisable or ionised substituents,
or
wherein the one or more dyes are provided in physiological solution at least doubly charged and/or preferably have at least three or four solubilising or ionisable or ionised substituents.

7. One or more polymethine dyes for use according to one of Claims 1 to 6, wherein the one or more dyes are selected from the group comprising:
DY630, DY631, DY632, DY633, DY634, DY635, DY636, DY647, DY648, DY649, DY650, DY651 and DY652, as well as DY590, DY548, DY495, DY405.

8. One or more polymethine dyes for use according to one of Claims 1 to 7 for monitoring an organ function following a liver or kidney transplant, for diagnosis of critically ill patients with sepsis or with multiple organ failure, or for preventative examination.

9. A method for acquiring data in order to determine an organ function *in vivo*/*ex vivo*, comprising the steps:
a) radiative excitation of one or more polymethine fluorescence dyes having a fluorescence emission maximum, measured in ethanol, of less than or equal to 820 nm as markers in blood, in a tissue or organ and/or in bile or urine, by means of a radiation source,
b) detection of the fluorescence emission of the one or more polymethine dyes by means of a detection device,
c) acquisition of the data of the measured fluorescence intensity by means of a data acquisition device,
wherein the organ, of which the function is determined, is the liver and/or kidney.

10. A method according to claim 9, wherein the dye is a benzopyrylium-based hemicyanine or an indodicarbocyanine chromophore.

11. A method according to Claim 9 or 10, wherein the one or more dyes are an asymmetrical polymethine having a substituted omega-(benz[b]pyran-4-yliden)alk-1-enyl) unit of the general structure 1: 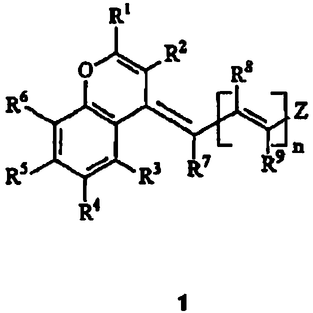 with Z as substituted benzoxazole-, benzothiazole-, 2,3,3-trimethylindolenine-, 2,3,3-trimethyl-4,5-benzo-3H-indolenine-, 2- and 4-picoline-, lepidine-, quinaldine- and 9-methylacridine- derivatives of the general formulas IIa or IIb or IIc 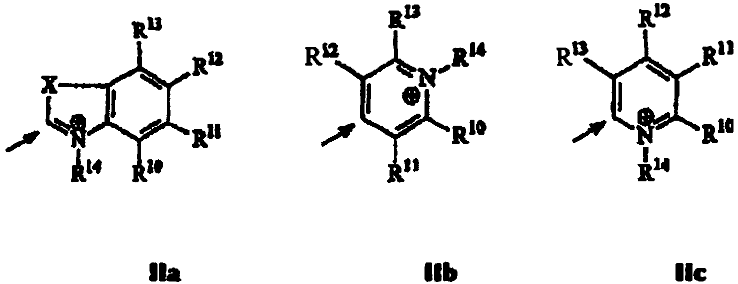 wherein
a. X stands for an element from the group O, S, Se or the structural element N-alkyl or C(alkyl)₂,
b. n stands for the numbers 1, 2 or 3,
c. R¹-R¹⁴ are the same or different and can be hydrogen, one or more alkyl, or aryl, heteroaryl, or heterocycloaliphatic residues, a hydroxyl group or alkoxy group, an alkyl-substituted or cyclic amine function, and/or can form two ortho residues, for example R¹⁰ and R¹¹, R⁴ and R⁵ and/or R⁶ and R⁵ together with a further aromatic ring,
d. at least one of the substituents R¹-R¹⁴ can represent a solubilising or ionisable or ionised substituent, such as cyclodextrin, sugar, SO₃⁻, PO₃²⁻, COO⁻, or NR₃⁺, which determines the hydrophilic properties of these dyes, wherein this substituent can also be bonded to the dye via a spacer group,
and
R¹ represents a substituent, which has a quaternary C atom, preferably t-butyl and adamantyl, in the alpha position at the pyran ring.

12. A method according to one of claims 9 to 11 for determining kidney function, wherein the one or more dyes are soluble in water but are insoluble in DMF or DMSO or for determining liver function, wherein the one or more dyes are soluble in DMF or DMSO and are insoluble in water.

13. A method according to one of Claims 9 to 12,
wherein the one or more dyes have a neutral charge in physiological solution or are provided at most singly charged and/or preferably have at most two solubilising or ionisable or ionised substituents,
or
wherein the one or more dyes are provided in physiological solution at least doubly charged and/or preferably have at least three or four solubilising or ionisable or ionised substituents.

14. A method according to one of claims 9 to 13, wherein the one or more dyes are selected from the group comprising:
DY630, DY631, DY632, DY633, DY634, DY635, DY636, DY647, DY648, DY649, DY650, DY651 and DY652, as well as DY590, DY548, DY495, DY405.

15. A method according to one of claims 9 to 14 for monitoring an organ function following a liver or kidney transplant, for diagnosis of critically ill patients with sepsis or with multiple organ failure, or for preventative examination.

16. A blood serum, bile or urine sample for use for acquiring data in order to determine an organ function, comprising one or more polymethine fluorescence dyes, according to one of Claims 1 to 8, having a fluorescence emission maximum, measured in ethanol, of less than or equal to 820 nm as markers, wherein the organ, the function of which is determined, is the liver and/or the kidney.

## Revendications

1. Un ou plusieurs colorants fluorescents de type polyméthine pour leur utilisation en tant que marqueurs aux fins de la collecte de données pour déterminer une fonction d'organe in vivo, dans lesquels les un ou plusieurs colorants fluorescents de type polyméthine présentent un maximum d'émission de fluorescence, mesuré dans l'éthanol, inférieur ou égal à 820 nm, et dans lesquels l'organe dont la fonction est déterminée est le foie et/ou les reins.

2. Un ou plusieurs colorants fluorescents de type polyméthine avec un maximum d'émission de fluorescence, mesuré dans l'éthanol, inférieur ou égal à 820 nm, pour leur utilisation dans un procédé servant à la collecte de données pour déterminer une fonction d'organe, comprenant les étapes de :
a) Excitation radiative in vivo de l'un ou de plusieurs colorants fluorescents de type polyméthine avec un maximum d'émission de fluorescence, mesuré dans l'éthanol, inférieur ou égal à 820 nm, en tant que marqueurs dans le sang, dans un tissu ou un organe et/ou dans la vésicule biliaire ou dans l'urine, avec une source de rayonnement,
b) Détection de l'émission de fluorescence des un ou plusieurs colorants fluorescents de type polyméthine avec un dispositif de détection,
c) Collecte des données de l'intensité de fluorescence mesurée avec un dispositif de collecte de données,
dans lesquels l'organe, dont la fonction est déterminée, est le foie et/ou les reins.

3. Un ou plusieurs colorants fluorescents de type polyméthine pour leur utilisation selon la revendication **1** ou **2**, dans lesquels le colorant est une hémicyanine à base de benzopyrylium ou un chromophore à base d'indodicarbocyanine.

4. Un ou plusieurs colorants fluorescents de type polyméthine pour leur utilisation selon l'une quelconque des revendications **1** à **3**, dans lesquels les un ou plusieurs colorants sont une polyméthine non symétrique, présentant un motif oméga-(benz[b]pyran-4-ylidène)alk-1-enyl) substitué de structure générale I 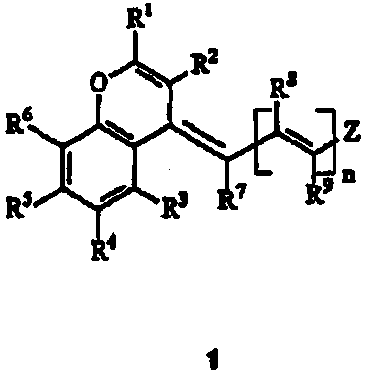 avec Z en tant que dérivés substitués de benzoxazole, de benzothiazole, de 2,3,3-triméthylindolénine, de 2,3,3-triméthyl-4,5-benzo-3H-indolénine, de 2- et 4-picoline, de lépidine, de quinaldine ainsi que de 9-méthylacridine des formules générales IIa ou IIb ou IIc 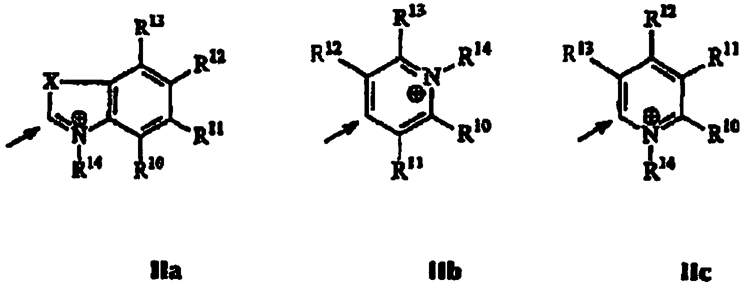 dans lesquelles
a. X représente un élément issu du groupe O, S, Se ou l'élément structurel N-alkyle ou C(alkyle)₂,
b. n représente les valeurs numériques 1, 2 ou 3,
c. R¹ - R¹⁴ sont identiques ou différents et peuvent être de l'hydrogène, un ou plusieurs alkyles ou des radicaux aryliques, hétéroaryliques ou hétérocycloaliphatiques, un groupe hydroxy ou alcoxy, une fonction amine alkyl-substituée ou cyclique et/ou deux radicaux en position ortho, par exemple R¹⁰ et R¹¹, R⁴ et R⁵ et/ou R⁶ et R⁵ peuvent former conjointement un autre cycle aromatique,
d. au moins un des substituants R¹ - R¹⁴ peut constituer un substituant solubilisant ou ionisable ou ionisé, tel que la cyclodextrine, le sucre, SO₃⁻, PO3⁻, COO⁻ ou NR₃⁺, qui détermine les propriétés hydrophiles desdits colorants, dans lesquels ledit substituant peut être rattaché au colorant également par l'intermédiaire d'un groupe espaceur, et
R¹ constitue un substituant, qui présente, en position alpha par rapport au cycle pyranne, un atome C quaternaire, de manière préférée est un t-butyle et un adamantyle.

5. Un ou plusieurs colorants fluorescents de type polyméthine pour leur utilisation selon l'une quelconque des revendications **1** à **4** pour la détermination d'une fonction des reins, dans lesquels les un ou plusieurs colorants sont solubles dans l'eau, mais sont insolubles dans du DMF (diméthylformamide) ou dans du DMSO (diméthylsulfoxyde)
ou
pour la détermination de la fonction du foie, dans lesquels les un ou plusieurs colorants sont solubles dans du DMF ou dans du DMSO et sont insolubles dans l'eau.

6. Un ou plusieurs colorants fluorescents de type polyméthine pour leur utilisation selon l'une quelconque des revendications **1** à **5**, dans lesquels les un ou plusieurs colorants présentent dans une solution physiologique une charge neutre ou sont présents au maximum avec une charge simple et/ou présentent de manière préférée au maximum deux substituants solubilisants ou ionisables ou ionisés
ou
dans lesquels les un ou plusieurs colorants sont présents dans une solution physiologique avec au moins une charge double et/ou présentent de manière préférée trois ou quatre substituants solubilisants ou ionisables ou ionisés.

7. Un ou plusieurs colorants fluorescents de type polyméthine pour leur utilisation selon l'une quelconque des revendications **1** à **6**, dans lesquels les un ou plusieurs colorants sont choisis parmi le groupe comprenant : DY630, DY631, DY632, DY633, DY634, DY635, DY636, DY647, DY648, DY649, DY650, DY651, DY652 ainsi que DY590, DY548, DY495, DY405.

8. Un ou plusieurs colorants de type polyméthine pour leur utilisation selon l'une quelconque des revendications **1** à **7** servant à surveiller une fonction d'organe après une transplantation du foie ou des reins, servant au diagnostic de patients malades dans un état critique dans le cas d'une septicémie ou dans le cas d'une insuffisance d'organe multiple ou aux fins d'un examen préventif.

9. Procédé de collecte de données pour déterminer une fonction d'organe in vitro/ex vivo, comprenant les étapes de :
a) Excitation radiative des un ou plusieurs colorants fluorescents de type polyméthine avec un maximum d'émission de fluorescence, mesuré dans l'éthanol, inférieur ou égal à 820 nm, en tant que marqueurs dans le sang, dans un tissu ou un organe et/ou dans la vésicule biliaire ou dans l'urine, avec une source de rayonnement,
b) Détection de l'émission de fluorescence des un ou plusieurs colorants de fluorescence de type polyméthine avec un dispositif de détection,
c) Collecte des données de l'intensité de fluorescence mesurée avec un dispositif de collecte de données,
dans lequel l'organe, dont la fonction est déterminée, est le foie et/ou les reins.

10. Procédé selon la revendication **9,** dans lequel le colorant est une hémicyanine à base de benzopyrylium ou un chromophore à base d'indodicarbocyanine.

11. Procédé selon la revendication **9** ou **10**, dans lequel les un ou plusieurs colorants sont une polyméthine non symétrique, présentant un motif oméga-(benz[b]pyran-4-ylidène)alk-1-enyl) substitué de structure générale I 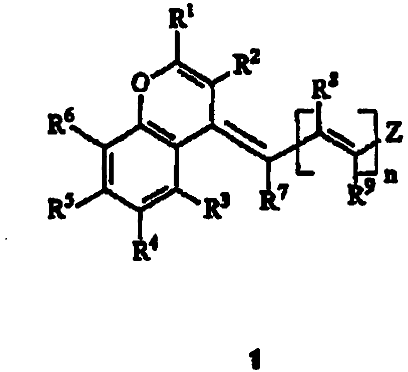 avec Z en tant que dérivés substitués de benzoxazole, de benzothiazole, de 2,3,3-triméthylindolénine, de 2,3,3-triméthyl-4,5-benzo-3H-indolénine, de 2- et 4-picoline, de lépidine, de quinaldine ainsi que de 9-méthylacridine des formules générales IIa ou IIb ou IIc 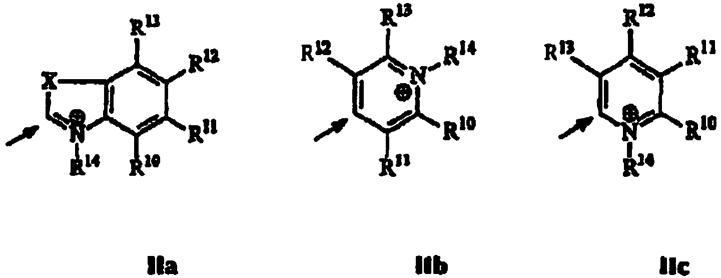 dans lesquelles
a. X représente un élément issu du groupe O, S, Se ou l'élément structurel N-alkyle ou C(alkyle)₂,
b. n représente les valeurs numériques 1, 2 ou 3,
c. R¹ - R¹⁴ sont identiques ou différents et peuvent être de l'hydrogène, un ou plusieurs alkyles ou des radicaux aryliques, hétéroaryliques ou hétérocycloaliphatiques, un groupe hydroxy ou alcoxy, une fonction amine alkyl-substituée ou cyclique et/ou deux radicaux en position ortho, par exemple R¹⁰ et R¹¹, R⁴ et R⁵ et/ou R⁶ et R⁵ peuvent former conjointement un autre cycle aromatique,
d. au moins un des substituants R¹ - R¹⁴ peut constituer un substituant solubilisant ou pouvant être ionisé ou ionisé, tel que la cyclodextrine, le sucre, SO₃⁻, PO₃⁻, COO⁻ ou NR₃⁺, qui détermine les propriétés hydrophiles desdits colorants, dans lequel ledit substituant peut être rattaché au colorant également par l'intermédiaire d'un groupe espaceur, et
R¹ constitue un substituant, qui présente, en position alpha par rapport au cycle pyranne, un atome C quaternaire, de manière préférée est un t-butyle et un adamantyle.

12. Procédé selon l'une quelconque des revendications **9** à **11** pour la détermination de la fonction des reins, dans lequel les un ou plusieurs colorants sont solubles dans l'eau, mais sont insolubles dans du DMF ou dans du DMSO
ou
pour la détermination de la fonction du foie, dans lequel les un ou plusieurs colorants sont solubles dans du DMF ou dans du DMSO et sont insolubles dans l'eau.

13. Procédé selon l'une quelconque des revendications **9** à **12**,
dans lequel les un ou plusieurs colorants présentent dans une solution physiologique une charge neutre ou sont présents au maximum avec une charge simple et/ou présentent de manière préférée au maximum deux substituants solubilisants ou ionisables ou ionisés
ou
dans lequel les un ou plusieurs colorants sont présents dans une solution physiologique avec au moins une charge double et/ou présentent de manière préférée trois ou quatre substituants solubilisants ou ionisables ou ionisés.

14. Procédé selon l'une quelconque des revendications **9** à **13**, dans lequel les un ou plusieurs colorants sont choisis parmi le groupe comprenant :
DY630, DY631, DY632, DY633, DY634, DY635, DY636, DY647, DY648, DY649, DY650, DY651, DY652 ainsi que DY590, DY548, DY495, DY405.

15. Procédé selon l'une quelconque des revendications **9** à **14** servant à surveiller une fonction d'organe après une transplantation du foie ou des reins, servant au diagnostic de patients malades dans un état critique dans le cas d'une septicémie ou dans le cas d'une insuffisance d'organe multiple ou aux fins d'un examen préventif.

16. Echantillon de sérum sanguin, de vésicule biliaire ou d'urine destiné à être utilisé aux fins de la collecte de données pour déterminer une fonction d'organe, comprenant un ou plusieurs colorants fluorescents de type polyméthine selon l'une quelconque des revendications **1** à **8** avec un maximum d'émission de fluorescence, mesuré dans de l'éthanol, inférieur ou égal à 820 nm en tant que marqueur/marqueurs, dans lequel l'organe, dont la fonction est déterminée, est le foie et/ou les reins.
